(19) Europäisches Patentamt
European Patent Office
Office européen des brevets

(11) EP 2 771 315 B1

(12) # FASCICULE DE BREVET EUROPEEN

(45) Date de publication et mention de la délivrance du brevet:
**13.04.2016 Bulletin 2016/15**

(21) Numéro de dépôt: **12799234.5**

(22) Date de dépôt: **26.10.2012**

(51) Int Cl.:
***C07C 269/04*** (2006.01) ***C07D 317/36*** (2006.01)
***C07D 317/34*** (2006.01) ***C07C 271/16*** (2006.01)
***C07F 11/00*** (2006.01)

(86) Numéro de dépôt international:
**PCT/FR2012/000437**

(87) Numéro de publication internationale:
**WO 2013/060950 (02.05.2013 Gazette 2013/18)**

(54) **PROCEDE DE PREPARATION D'UN COMPOSE COMPRENANT AU MOINS UN MOTIF béta-HYDROXY-URETHANE ET/OU AU MOINS UN MOTIF gamma-HYDROXY-URETHANE.**

VERFAHREN ZUR HERSTELLUNG EINER VERBINDUNG MIT MINDESTENS EINER -HYDROXY-URETHANEINHEIT UND/ODER MINDESTENS EINER gamma-HYDROXY-URETHANEINHEIT

METHOD FOR PREPARING A COMPOUND COMPRISING AT LEAST ONE beta-HYDROXY-URETHANE UNIT AND/OR AT LEAST ONE gamma-HYDROXY-URETHANE UNIT

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **28.10.2011 FR 1159818**

(43) Date de publication de la demande:
**03.09.2014 Bulletin 2014/36**

(73) Titulaires:
- **Société d'Exploitation Générale de Produits Industriels - S.E.G (SAS)**
**34560 Poussan (FR)**
- **Centre National de la Recherche Scientifique (Etablissement Public)**
**75794 Paris (FR)**
- **Ecole Nationale Supérieure de Chimie de Montpellier**
**34296 Montpellier (FR)**
- **Université Montpellier 2, Sciences et Techniques**
**34095 Montpellier Cedex 5 (FR)**

(72) Inventeurs:
- **SOULES, Aurélien**
**34310 Capestang (FR)**
- **CAILLOL, Sylvain**
**34090 Montpellier (FR)**
- **JOUBERT, Jean-Paul**
**34000 Montpellier (FR)**
- **MARTINS, José**
**34110 Frontignan (FR)**
- **BOUTEVIN, Bernard**
**34090 Montpellier (FR)**

(74) Mandataire: **Roman, Alexis**
**Cabinet Roman**
**35, rue Paradis**
**B.P. 30064**
**13484 Marseille Cedex 20 (FR)**

(56) Documents cités:
**EP-A1- 0 065 026** **WO-A1-98/55451**
**US-A- 4 268 684** **US-A- 5 347 034**
**US-B2- 6 410 778**

- **DATABASE WPI Week 198008 Thomson Scientific, London, GB; AN 1980-13739C XP002683744, & JP 55 004316 A (MITSUBISHI CHEM IND LTD) 12 janvier 1980 (1980-01-12)**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 2 771 315 B1

## Description

### Domaine technique de l'invention.

L'invention a pour objet un procédé de préparation d'un composé comprenant au moins un motif β-hydroxy-uréthane et/ou au moins un motif γ-hydroxy-uréthane, consistant à faire réagir un composé A comprenant au moins un motif réactif cyclocarbonate avec un composé B comprenant au moins un motif réactif amino ($-NH_2$) en présence d'un catalyseur. Elle a également pour objet l'utilisation d'un catalyseur comprenant au moins un complexe organométallique et un co-catalyseur choisi dans le groupe des bases de Lewis et/ou des sels de tétra-alkyl ammonium, pour catalyser le procédé selon l'invention.

Elle concerne le domaine technique de préparation de dérivés d'uréthane.

### État de la technique.

Les polyuréthanes occupent une place de choix dans le domaine des polymères, en raison à la fois de leur nature très diversifiées et de leurs application dans de nombreux produits tels que les revêtements, les peintures, les adhésifs, les vernis, les mousses, les fibres élastiques, les matériaux isolants, les résines diélectriques, les gaines de câbles électriques ou encore les dispositifs médicaux.

Les polyuréthanes conventionnels sont habituellement synthétisés par réaction de polycondensation entre un di- ou polyol et un di- ou polyisocyanate. La toxicité et l'instabilité des isocyanates constituent un inconvénient majeur. En outre, les isocyanates sont classiquement obtenus à partir du phosgène, lui-même un réactif dangereux pour la santé et l'environnement.

De nombreux travaux de recherche ont été effectués pour mettre en oeuvre des procédés moins nocifs. Ainsi, depuis quelques années, un nouveau procédé de préparation de polyuréthanes est étudié. Il est apparu que la réaction de condensation entre une diamine et une molécule comportant deux groupes cyclocarbonates conduit à la formation de polyuréthanes, sans faire appel à des composés isocyanates (Ochia, B; Satoh, Y; Endo, T, J, Polym. Sci. Part A : Polym Chem., 39, 4091, 2001). Un tel procédé est par exemple décrit dans les documents de brevets suivants: US5340889 (Crawford et al.), US2802022 (Groszos et al.), US4758615 (Engel et al.), US6120905 (Figovsky), US2935494 (Whelan et al.), US3072613 (Whelan et al.), US3072613 (Whelan et al.), EP1070733 (POLYMATE LTD et al.). L'intérêt de ce nouveau procédé est qu'il conduit à des polyuréthanes sans isocyanates présentant une meilleure stabilité hydrolytique que les polyuréthanes conventionnels du fait de la formation d'un groupement hydroxyle (-OH) en position β de la fonction uréthane (ou carbamate) [Figovski, 0., Improving the protective properties of non metallic corrosion resistant materials and coatings. Journal of Medeleev Chemical Society, N. Y., USA 1988 Vol 33 No 3. pp 31-36]. Cependant, l'énergie d'activation de la réaction étant très élevée, une conversion quantitative des réactifs est atteinte lorsque la polycondensation est réalisée à des températures supérieures à 80°C et lorsque le temps de la réaction est supérieur à 200h.

Afin d'optimiser cette réaction de condensation, diverses méthodes préconisent l'emploi de catalyseurs. Parmi ces méthodes, on peut citer celles décrites dans les documents de brevets US5055542 (Honel et al.), US5132458 (Honel et al.), US5977262 (Anderson), WO2003066580 (UCB, S.A.), WO2005016993 (HUNTSMAN ADVANCED MATERIALS GMBH), US4268684 (Arthur G. et al.), EP0065026 (DOW CHEMICAL Co). Les catalyseurs utilisés sont généralement choisis parmi les sels de métaux, bases de Lewis, les sels alcalins, les sels d'ammonium quaternaires et les argiles modifiées. Mais la température et la durée de réaction restent élevées, ce qui est très pénalisant au niveau industriel.

Il existe donc un besoin pour une méthode efficace de synthèse d'hydroxyuréthanes, en particulier de composés comprenant au moins un motif β-hydroxy-uréthane et/ou au moins un motif γ-hydroxy-uréthane.

Ainsi, un premier objectif de la présente invention est de proposer un procédé de préparation de composés comprenant au moins un motif β-hydroxy-uréthane et/ou au moins un motif γ-hydroxy-uréthane, qui soit significativement plus efficace que les procédés connus de l'art antérieur.

Un autre objectif de l'invention est de proposer un procédé de préparation de composés comprenant au moins un motif β-hydroxy-uréthane et/ou au moins un motif γ-hydroxy-uréthane, qui puisse être réalisé avec un rendement élevé, répondant ainsi aux exigences économiques de l'industrie.

L'invention a également pour objectif de proposer un procédé de préparation de composés comprenant au moins un motif β-hydroxy-uréthane et/ou au moins un motif γ-hydroxy-uréthane, qui puisse être réalisé dans des conditions moins sévères que les procédés connus jusqu'à présent, notamment en ce qui concerne la température de réaction.

L'invention a également pour objectif de proposer un procédé qui permet de conduire en un temps de réaction très court à des composés comprenant au moins un motif β-hydroxy-uréthané et/ou au moins un motif γ-hydroxy-uréthane, de meilleure qualité que ceux obtenus par les procédés connus de l'art antérieur.

Encore un autre objectif de l'invention est de proposer un procédé de préparation de composés comprenant

au moins un motif β-hydroxy-uréthane et/ou au moins un motif γ-hydroxy-uréthane, qui vise à mieux préserver la santé des opérateurs et à mieux respecter l'environnement.

**Divulgation de l'invention.**

Il a à présent été trouvé que ces buts peuvent être atteints en totalité ou en partie au moyen du procédé qui est décrit ci-après.

La solution proposée par l'invention est un procédé de préparation d'un composé comprenant au moins un motif β-hydroxy-uréthane et/ou au moins un motif γ-hydroxy-uréthane, consistant à faire réagir un composé A comprenant au moins un motif réactif cyclocarbonate avec un composé B comprenant au moins un motif réactif amino ($-NH_2$) en présence d'un catalyseur. Ce procédé est remarquable en ce que le catalyseur comprend au moins un complexe organométallique et un co-catalyseur choisi dans le groupe des bases de Lewis et/ou des sels de tétra-alkyl ammonium.

De manière inattendue, la demanderesse a découvert lors de ses recherches qu'en utilisant un catalyseur comprenant au moins un complexe organométallique combiné à un co-catalyseur, on pouvait optimiser de manière remarquable le procédé de préparation de composés comprenant au moins un motif β-hydroxy-uréthane et/ou au moins un motif γ-hydroxy-uréthane.

En effet, l'utilisation d'un tel catalyseur permet:

- de réaliser le procédé de l'invention à des températures inférieures à 170°C, le plus souvent inférieures à 100°C, de préférence inférieures à 50°C, encore préférentiellement à la température ambiante (environ 25°C). En particulier, dans certains cas, notamment lorsque le composé A et/ou le composé B est faiblement réactif (par ex. la 3-aminométhyl-3,5,5-triméthyl cyclohexylamine, les polyamine masquées de la famille des polycétimines, ou autres) ou présente un caractère oligomérique ou polymérique, le procédé de l'invention peut, pour répondre au besoin d'homogénéisation du mélange réactionnel ou de déblocage d'amines masquées, être conduit à des températures supérieures à la température ambiante et inférieures à 100°C, en particulier supérieures à la température ambiante et inférieures à 170°C.
- d'augmenter la cinétique de réaction de manière significative, et
- d'accéder aux composés comprenant au moins un motif β-hydroxy-uréthane et/ou au moins un motif γ-hydroxy-uréthane avec un très bon rendement et de très bonne qualité, et ce en un temps de réaction beaucoup plus court qu'avec les catalyseurs préconisés par les méthodes connues, ce temps de réaction étant généralement inférieur à 600 minutes, le plus souvent inférieur à 480 minutes, et dans certains cas ce temps de réaction peut être de quelques minutes seulement, voire même de quelques secondes.

Par ailleurs, un tel procédé ne nécessite pas l'utilisation de produits toxiques tels que les isocyanates et peut être mis en oeuvre sans solvant ou avec peu de solvant lorsque cela est nécessaire.

Le procédé selon l'invention possède donc un potentiel important.

D'autres caractéristiques préférées de l'invention sont listées ci-dessous, chacune de ces caractéristiques pouvant être considérée seule ou en combinaison avec les caractéristiques remarquables définies ci-dessus:

- le complexe organométallique contient un métal alcalin, alcalino-terreux ou de transition choisi parmi les groupes IA, IIA, IIIA, IIIB, IVA, IVB, VB, VIB, VIIB et VIII du tableau périodique, et au moins deux ligands β-dicétonate.

- le complexe organométallique est choisi dans la liste suivante: tris(acétylacétonate) d'aluminum(III), tris(hexafluoroacétylacétonate) d'aluminium(III), tris(triftuoroacétylacétonate) d'aluminium(III), tris(2,2,6,6-tétraméthyl-3,5-heptanedionato)aluminium(III), bis(acétylacétonate) de calcium(II), tris(acétylacétonate) de chrome(III), tris(2,2,6,6-tétraméthyl-3,5-heptanedionate) de chrome(III), tris(acétylacétonate) de cobalt(III), tris(nitro-acétylacétonate) de cobalt(III), tris(2,2,6,6-tétraméthyl-3,5-heptanedionate) de Cobalt(III), bis(acétylacétonate) de cuivre(II), bis(2,2,6,6-tétraméthyl -3,5-heptanedionate) de cuivre(II), tris(acétylacétonato)gallium (III), acétylacétonate d'hafnium(IV), tris(acétylacétonato)indium(III), tris(acétylacétonate) de fer(III), tris(2,2,6,6- tétraméthyl -3,5-heptanedionate) de fer(III), tris(acétylacétonate) de manganèse (III), bis(acétylacétonate) de nickel(II), bis(acétylacétonate) de palladium(II), bis(trifluoroacétylacétonate) de palladium(II), acétylacétonate de sodium, bis(acétylacétonate) d'oxyde de titane (IV), bis(2,2,6,6-tétraméthyl-3,5-heptànedionate) d'oxyde de titane(IV), tris(2,2,6,6-tétraméthyl-3,5-heptanedionato) de titane (III), tris(2,2,6,6-tétraméthyl-3,5-heptanedionato) oxotitane (IV), dichloro-bis-(2,2,6,6-tétraméthyl-3,5-heptanedionato)titanium(IV), le (diisopropoxyde)bis(acétylacétonate) de titane(IV), di(isopropoxide)bis(2,2,6,6-tétraméthyl-3,5-heptanedionato)titanium(IV), bis(acétylacétonate) de zinc(II), tetrakis(acétylacétonate)zirconium (IV), tetrakis(hexafluoroacétylacétonate) de zirconium(IV), tetrakis(2,2,6,6-tétraméthyl-3,5-heptanedionato)zirconium(IV), tetrakis(trifluoroacétylacétonate) de zirconium(IV).

- le rapport molaire complexe organométallique/composé A est compris entre 0,001 à 0,05, de préférence entre 0.002 et 0,01.

- le catalyseur comprend en outre un co-catalyseur choisi parmi les trialkylamines, les amines hétérocycliques aromatiques, les trialkylphosphines, les triarylphosphines, les trialkylphosphites, les triarylphosphites, les sels de tetralakyl ammoniums ou leurs mélanges.

- Le co-catalyseur est utilisé à raison de 1,5 à 3 moles par mole de complexe organométallique, de préférence à 2 moles par mole de complexe organométallique.

- le composé A est choisi dans le groupe des composés, oligomères ou polymères terminés par au moins un cyclocarbonate, de préférence au moins deux cyclocarbonates.

- le composé **A** est un composé répondant à:

  - la formule générale **A1**:

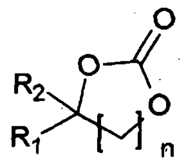

**A1**

dans laquelle :

  - n représente un nombre entier égal à 1 ou 2,

  - $\mathbf{R_1}$ représente un atome d'hydrogène ou un groupe alkyle en $C_{1-6}$, linéaire ou ramifié, saturé ou insaturé, et le cas échéant, substitué par un ou plusieurs substituants choisis parmi halogène, hydroxy, cyano, carboxy, trifluorométhyl, alkyl en $C_{1-6}$, alkylamino en $C_{1-6}$, dialkylamino en $C_{1-6}$, alcoxy en $C_{1-6}$, alkylcarboxylate en $C_{1-6}$,

  - $\mathbf{R_2}$ représente un atome d'hydrogène ou un groupe choisi parmi : un alkyle en $C_{1-20}$, linéaire ou ramifié, saturé ou insaturé, et dans lequel des unités méthylènes non voisines peuvent être remplacées par des radicaux -O-, -S-,-S(O)-, $-SO_2-$, -O-C(=O)-, $-N(R^{21})-$, $-N(R^{21})-C(=O)-$, $-N(R^{21})-C(=O)-O-$, $-N(R^{21})-C(=O)-N(R^{22})-$, avec $R^{21}$ et $R^{22}$ identiques ou différents, représentant des atomes d'hydrogène ou des radicaux choisis parmi alkyl en $C_{1-6}$ ou aryle en $C_{6-14}$; un cycloalkyle en $C_{3-10}$; un aryle en $C_{6-14}$; un hétérocyle en $C_{3-10}$ portant un ou plusieurs hétéroatomes choisis parmi N, O, S; un hétéroaryl en $C_{4-13}$ portant un ou plusieurs hétéroatomes choisis parmi N, O, S ; les membres de ce groupe étant éventuellement substitués par un ou plusieurs substituants choisis parmi halogène, hydroxy, cyano, carboxy, trifluorométhyl, alkyl en $C_{1-6}$, alkylamino en $C_{1-6}$, dialkylamino en $C_{1-6}$, alcoxy en $C_{1-6}$, alkylcarboxylate en $C_{1-6}$,

  - ou, la formule générale **A2 :**

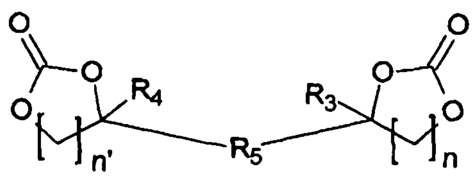

**A2**

dans laquelle :

- n, n', identiques ou différents représentent un nombre entier égal à 1 ou 2,

- **$R_3$** et **$R_4$,** identiques ou différents, représentent un atome d'hydrogène ou un groupe alkyle en $C_{1-6}$, linéaire ou ramifié, saturé ou insaturé, et le cas échéant, substitués par un ou plusieurs substituants choisis parmi halogène, hydroxy, cyano, carboxy, trifluorométhyl, alkyl en $C_{1-6}$, alkylamino en $C_{1-6}$, dialkylamino en $C_{1-6}$, alcoxy en $C_{1-6}$, alkylcarboxylate en $C_{1-6}$,

- **$R_5$** représente:

  - une liaison directe,

  - un groupe bivalent choisi parmi: : un alkylène en $C_{1-20}$, linéaire ou ramifié, saturé ou insaturé, et dans lequel des unités méthylènes non voisines peuvent être remplacées par des radicaux -O-, -S-, -S(O)-, $-SO_2-$, -C(=O)-O-,-N($R^{21}$)-, -N($R^{21}$)-C(=O)-, -N($R^{21}$)-C(=O)-O-, -N($R^{21}$)-C(=O)-N($R^{22}$)-, avec $R^{21}$ et $R^{22}$ identiques ou différents, représentant des atomes d'hydrogène ou des radicaux choisis parmi alkyl en $C_{1-6}$ ou aryle en $C_{6-14}$; un cycloalkylène en $C_{3-10}$; un arylène en $C_{6-14}$; un hétérocyclène en $C_{3-10}$ portant un ou plusieurs hétéroatomes choisis parmi N, O, S; un hétéroarylène $C_{4-13}$ portant un ou plusieurs hétéroatomes choisis parmi N, O, S; les membres de ce groupe étant éventuellement substitués par un ou plusieurs substituants choisis parmi halogène, hydroxy, cyano, carboxy, trifluorométhyl, alkyl en $C_{1-6}$, alkylamino en $C_{1-6}$, dialkylamino en $C_{1-6}$, alcoxy en $C_{1-6}$, alkylcarboxylate en $C_{1-6}$,

  - ou, un groupe bivalent présentant la formule générale **I** suivante:

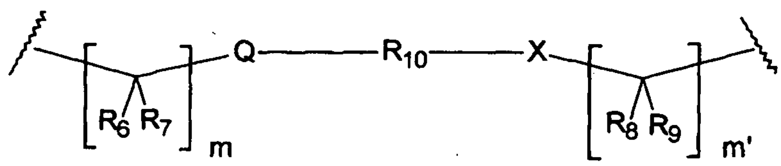

**(I)**

dans laquelle :

- m et m' identiques ou différents désignent un nombre entier compris entre 1 à 100,

- **$R_6$, $R_7$, $R_8$** et **$R_9$,** identiques ou différents, représentent des atomes d'hydrogène ou des radicaux alkyles en $C_{1-6}$, linéaires ou ramifiés, saturés ou insaturés, et le cas échéant, substitués par un ou plusieurs substituants choisis parmi halogène, hydroxy, cyano, carboxy, trifluorométhyl, alkyl en $C_{1-6}$, alkylamino en $C_{1-6}$, dialkylamino en $C_{1-6}$, alcoxy en $C_{1-6}$, alkylcarboxylate en $C_{1-6}$,

- **Q** et **X** identiques ou différents représentent un groupe -O-C(=O)-,-N($R^{21}$)-, -N($R^{21}$)-C(=O)-, -N($R^{21}$)-C(=O)-O-, -N($R^{21}$)-C(=O)-N($R^{22}$)-, avec $R^{21}$ et $R^{22}$ sont tels que définis précédemment,

- **$R_{10}$** représente un groupe bivalent choisi parmi : un alkylène en $C_{1-20}$, linéaire ou ramifié, saturé ou non, et dans lequel des unités méthylènes non voisines peuvent être remplacées par des radicaux -O-, -S-, -S(O)- $-SO_2-$,-C(=O)-O-, -N($R^{21}$)-, -N($R^{21}$)-C(=O)-, -N($R^{21}$)-C(=O)-O-, -N($R^{21}$)-C(=O)-N($R^{22}$)-, avec $R^{21}$ et $R^{22}$ sont tels que définis précédemment; un cycloalkylène en $C_{3-10}$; un arylène en $C_{6-14}$; un hétérocyclène en $C_{3-10}$ portant un ou plusieurs hétéroatomes choisis parmi N, O, S; un hétéroarylène $C_{4-13}$ portant un ou plusieurs hétéroatomes choisis parmi N, O, S; les membres de ce groupe bivalent étant éventuellement substitués par un ou plusieurs substituants choisis parmi halogène, hydroxy, cyano, carboxy, trifluorométhyl, alkyl en $C_{1-6}$, alkylamino en $C_{1-6}$, dialkylamino en $C_{1-6}$, alcoxy en $C_{1-6}$, alkylcarboxylate en $C_{1-6}$;

- ou, un groupe répondant à l'une des formules **II** ou **III:**

$-(CH_2-CH_2-O)_t-CH_2-CH_2-$ **(II),** ou

$$-C(=O)-CH_2-O-(CH_2-CH_2-O)_{t'}-CH2-C(=O)- \quad \textbf{(III)}$$

dans lesquelles :

- **t, t',** identiques ou différents, désignent un nombre entier compris entre 1 et 100.

  • le composé **A est** un composé **A3** obtenu par :

- la réaction du 4-hydroxyméthyl-1,3-dioxolan-2-one avec un composé comprenant une ou plusieurs fonctions isocyantes,
- la réaction d'estérification du 4-hydroxyméthyl-1,3-dioxolan-2-one avec un composé comprenant une ou plusieurs fonctions acides carboxyliques,
- la réaction du triglycidyl isocyanurate avec le dioxyde de carbone, ou
- la réaction d'un composé comprenant deux ou plusieurs groupes éthers de glycidyle avec le dioxyde de carbone.

• le composé **B** est choisi dans le groupe des composés, oligomères ou polymères terminés par au moins un motif réactif ($-NH_2$), de préférence par au moins deux motifs réactifs ($-NH_2$).

• le ou les motifs réactifs ($-NH_2$) sont portés par des carbones saturés (sp3), lesquels sont également porteurs d'au moins un hydrogène, de préférence de deux hydrogènes.

• le composé **B** comprenant au moins un motif réactif amino ($-NH_2$) est choisi dans la liste suivante: butylamine ; hexylamine; cyclohexylamine; éthanotamine; propanolamine; éthylène diamine ; propylène diamine; butylène diamine ; pentaméthylène diamine ; hexaméthylène diamine; heptaméthylène diamine ; tétraméthylène diamine ; octaméthylène diamine; nonaméthylène diamine ; décaméthylène diamine ; méthyl-2-pentaméthylène diamine ; undécaméthylène diamine ; dodécaméthylène diamine ; xylylène diamine; isophorone diamine ; triméthyl hexaméthylène diamine ; 1,2-diaminocyclohexane ; 1,4-diaminocyclohexane; 4,4'-diaminocyclohexyl méthane; 2-(2-aminoéthoxy)éthanol; bis-(3-méthyl4-aminocyclohexyl)méthane; 2,2-bis-(4-aminocyclohexyl)propane; nitrile tri(éthaneamine); bis-(3-aminopropyl)méthylamine; 3-amino-1-(méthylamino)propane; 3-amino-1-(cyclohexylamino)propane; N-(2-hydroxyéthyl)éthylène diamine; 4,7-dioxadécane-1,10-diamine; 4,9-dioxadodécane-1,12-diamine; 7-méthyl 4,10-dioxatridécane-1,1, 3-diamine; une polyéther monoamine de préférence choisie parmi l'hydroxy polyéthyléne glycol amine et la méthoxypolyéthyléne glycol amine, une polyéther diamine, de préférence choisie parmi une polyoxyéthylène diamine et une polyoxypropylène diamine ; et/ou un oligomère ou polymère terminé par au moins un motif réactif amino ($-NH_2$), de préférence par deux ou plusieurs motifs réactifs amino ($-NH_2$), ledit oligomère ou polymère pouvant avoir un squelette polyamide, polyéther et/ou polyester.

• le composé **A** et le composé **B** sont mélangés de manière à ce que le rapport molaire composé **B** sur composé **A** soit compris entre 0,5 et 2, de préférence égal à 1.

• le composé **A** et le composé **B** sont préalablement mélangés avec un plastifiant avant la mise en contact avec le catalyseur comprenant au moins un complexe organométallique, éventuellement associé à un co-catalyseur.

• Le procédé est opéré à une température comprise entre 0°C et 170°C, de préférence 0°C et 100°C, encore préférentiellement à la température ambiante.

Un autre aspect de l'invention concerne l'utilisation d'au moins un complexe organométallique contenant un métal alcalin, alcalino-terreux ou de transition choisi parmi les groupes IA, IIA, IIIA ; IIIB, IVA, IVB, VB, VIB, VIIB et VIII du tableau périodique, et au moins deux ligands acétylacétonato, et éventuellement associé à un co-catalyseur, pour catalyser la réaction de condensation entre un composé **A** comprenant au moins un motif réactif cyclocarbonate et un composé **B** comprenant au moins un motif réactif amino ($-NH_2$).

**Description des figures.**

D'autres avantages et caractéristiques de l'invention apparaîtront mieux à la lecture de la description d'un mode de réalisation préféré qui va suivre, en référence aux figures annexés, réalisées à titre d'exemples indicatifs et non limitatifs et sur lesquels :

- la figure 1 illustre un spectre FTIR réalisé à 25 secondes sur le brut réactionnel de la réaction de condensation entre la triméthylhexaméthylène diamine et le 4-(hydroxyméthyl)-1,3-dioxolan-2-one en présence d'une quantité catalytique de la triéthylamine;

- la figure 2 illustre le suivi, par spectroscopie FTIR, de l'évolution de la réaction de condensation entre la triméthylhexaméthylène diamine et le 4-(hydroxyméthyl)-1,3-dioxolan-2-one catalysée par un catalyseur selon l'invention ou par certains catalyseurs préconisés dans les méthodes connues de l'art antérieur ;

- la figure 3 illustre le suivi, par spectroscopie FTIR, de l'évolution de la réaction de condensation entre la triméthylhexaméthylène diamine et l'acétate (2-oxo-1,3-dioxolan-4-yl)méthyle catalysée par le catalyseur comprenant le tris(2,4-pentanedione)chrome(III) associé à la triéthylamine. (Superposition des spectres FTIR réalisés sur des prélèvements du mélange réactionnel à 25 secondes et 180 secondes).

- la figure 4 illustre le suivi, par spectroscopie FTIR, de l'évolution de la réaction de condensation entre la tétraéthylène pentamine et le 4-(hydroxyméthyl)-1,3-dioxolan-2-one catalysée, à température ambiante, par le catalyseur comprenant le tris(2,4-pentanedione)chrome(III) associé à la triéthylamine.

- la figure 5 illustre le suivi, par spectroscopie FTIR, de l'évolution de la réaction de condensation entre la 3-aminométhyl-3,5,5-triméthylcyclohexylamine et le 4-(hydroxyméthyl)-1,3-dioxolan-2-one catalysée, à température ambiante, par le catalyseur comprenant le tris(2,4-pentanedione)chrome(III) associé à la triéthylamine.

- la figure 6 illustre le suivi, par spectroscopie FTIR, de l'évolution de la réaction de condensation entre la Jeffamine® D230 et le 4-(hydroxyméthyl)-1,3-dioxolan-2-one catalysée, à température ambiante, par le catalyseur comprenant le tris(2,4-pentanedione)chrome(III) associé à la triéthylamine.

- la figure 7 illustre le suivi, par spectroscopie FTIR, de l'évolution de la réaction de condensation entre la tetraéthylènepentamine et un composé comprenant deux motifs réactifs cyclocarbonates obtenu par la réaction du 4-(hydroxyméthyl)-1,3-dioxolan-2-one avec le diisocyante Desmodur N3400, la réaction de condensation étant catalysée, à température ambiante, par le catalyseur comprenant le tris(2,4-pentanedione)chrome(III) associé à la triéthylamine.

## Modes de réalisation de l'invention.

Dans le cadre de la présente invention, et sauf mention différente dans le texte, on entend par :

- « atome d'halogène » : un fluor, un chlore, un brome ou un iode, de préférence un atome de fluor ;

- « alkyle» : un groupe aliphatique saturé ou non, linéaire ou ramifié ayant 1 à 20 atomes de carbone successifs (abrégé par alkyle en $C_{1-20}$). A titre d'exemples, on peut citer les groupes méthyle, éthyle, propyle, isopropyle, butyle, isobutyle, tertbutyle, pentyle, hexyle, heptyle, octyle, décyle, undécyle, dodécyle, tridécyle, tetradécyle, pentadécyle, hexadécyle, heptadécyle, octadécyle, nonadécyl, icosanyle, propényle, butényle, pentényle, 1-hexényle, heptènyle, octènyle, nonènyle, décènyle, undécènyle, oléyle, linolyle et linolényle etc. Sauf dans les cas où il est spécifié, le radical alkyle peut comporter 1 à 6 atomes de carbones successifs (abrégé par alkyle en $C_{1-6}$). Les groupes alkyles peuvent éventuellement être substitués par un groupe aryle tel que défini ci-après, auquel cas on parle de groupe arylalkyle. Des exemples de groupes arylalkyle sont notamment benzyle, phénéthyle, phénylpropyle, etc.

- « alkylène » : un groupe alkyle tel que précédemment défini, divalent.

- « cycloalkyle » : un groupe alkyle cyclique monovalent ayant 3 à 10 atomes de carbone, saturé ou insaturé. A titre d'exemples, on peut citer les groupes cyclopropyle, méthylcyclopropyle, cyclobutyle, cyclopentyle, cyclohexyle, cyclooctyle, cyclononyle, cyclodécyle, etc.

- « cycloalkylène » : un groupe cycloalkyle tel que précédemment défini, divalent.

- « aryle » : un groupe hydrocarboné aromatique, ayant 6 à 14 atomes de carbone (abrégé par aryle en $C_{3-14}$), encore plus préférentiellement 6 atomes de carbone. Les groupes aryles incluent notamment les radicaux phényle, naphtyle et bi-phényle.

- « arylène » : un groupe aryle tel que précédemment défini, divalent.

- « hétérocycle » : un groupe saturé ou insaturé (non aromatique) mono- ou bi-cyclique éventuellement fusionné ou ponté comportant de 3 à 10 atomes (abrégé par hétérocycle en $C_{3-10}$) dont un au moins atome est choisi parmi les atomes d'oxygène, d'azote ou de soufre,. On citera par exemple les groupes 2,3-dihydrobenzofurane et 1,4-benzodioxane, phthalimide, etc.

- « hétérocyclène » un groupe hétérocyclyle tel que précédemment défini, divalent.

- « hétéroaryle » : un groupe aromatique mono- ou bi-cyclique comprenant entre 4 et 13 atomes de carbone et comprenant entre 1 et 3 hétéroatomes, tels que l'azote, l'oxygène ou le soufre. A titre d'exemples de groupes hétéroaryles, on peut citer les groupes : furane, pyridine, thiazole, imidazole benzothiophène etc.

- « hétéroarylène » un groupe hétéroaryle tel que précédemment défini, divalent.

- « alcoxy » : un groupe -O-alkyle où le groupe alkyle est tel que précédemment défini et a 1 à 6 atomes de carbones (abrégé alcoxy en $C_{1-6}$).

- « alkylamino » : un groupe -NH-alkyle où le groupe alkyle est tel que précédemment défini et a 1 à 6 atomes de carbones (abrégé par alkylamino en $C_{1-6}$).

- « dialkylamino » : un groupe $-N(alkyle)_2$ où le groupe alkyle est tel que précédemment défini et a 1 à 6 atomes de carbones (abrégé par dialkylamino en $C_{1-6}$).

- « alkylcarboxylate » un groupe -O-C(=O)-alkyle où le groupe alkyle est tel que précédemment défini et a 1 à 6 atomes de carbones (abrégé par alkylcarboxylate en $C_{1-6}$).

Les groupes alkyle, alkylène, cycloalkyle, cycloalkylène, aryle , arylène, hétérocycle, hétérocyclène, hétéroaryle, et hétéroarylène peuvent le cas échéant être substitués par un ou plusieurs substituants choisis parmi halogène, hydroxy, cyano, carboxy, trifluorométhyl, alkyl en $C_{1-6}$, alkylamino en $C_{1-6}$, dialkylamino en $C_{1-6}$, alcoxy en $C_{1-6}$, alkylcarboxylate en $C_{1-6}$.

« composé comprenant au moins un motif β-hydroxy-uréthane et/ou au moins un motif γ-hydroxy-uréthane » : un composé comprenant un ou plusieurs motifs β-hydroxy-uréthane; ou un ou plusieurs motifs γ-hydroxy-uréthane; ou un ou plusieurs motifs β-hydroxy-uréthane et un ou plusieurs motifs γ-hydroxy-uréthane.

L'invention est basée sur la découverte que la présence d'un catalyseur à base de complexes organométalliques, éventuellement associés à un co-catalyseur, permet d'accélérer la formation de composés comprenant au moins un motif β-hydroxy-uréthane et/ou au moins un γ-hydroxy-uréthane dans des conditions douces et avec un excellent taux de conversion.

Les complexes organométalliques convenant à la mise en oeuvre du procédé de l'invention sont ceux contenant un métal alcalin, alcalino-terreux ou de transition choisi parmi les groupes IA, IIA, IIIA ; IIIB, IVA, IVB, VB, VIB, VIIB et VIII du tableau de classification périodique, et au moins deux ligands β dicétonate.

Comme exemples de métal de transition impliqué dans le complexe organométallique, on peut citer l'antimoine, l'argent, le cadmium, le chrome, le cobalt, le cuivre, le étain, le fer, le gallium, le germanium, l'hafnium, l'indium, l'iridium, le manganèse, le mercure, le molybdène, le nickel, le niobium, l'or, l'osmium, le palladium, le platine, le rhénium, le rhodium, le ruthénium, le scandium, le tantale, le titane, le technétium, le tungstène, le vanadium, l'yttrium, le zinc, et le zirconium.

Les β-dicétonates servant de ligands dans le complexe organométallique sont de préférence choisis parmi l'acétylacétonato, le 1,1,1,5,5,5-hexa-fluoro-acétylacétonate, le 1,1,1-trifluoro-acétylacétonate, 1,1,1-triftuoro-5,5-di-méthylacétylacétonate et le 2,2,6,6-tetraméthyl-3,5-heptanedionate.

De préférence, le complexe organométallique est choisi dans la liste suivante : tris(acétylacétonate) d'aluminum(III), tris(hexafluoroacétylacétonate) d'aluminium(III), tris(trifluoroacétylacétonate) d'aluminium(III), tris(2,2,6,6-tétraméthyl-3,5-heptanedionato)aluminium(III), bis(acétylacétonate) de calcium(II), tris(acétylacétonate) de chrome(III), tris(2,2,6,6-tétraméthyl-3,5-heptanedionate) de chrome(III), tris(acétylacétonate) de cobalt(III), tris(nitro-acétylacétonate) de cobalt(III), tris(2,2,6,6-tétraméthyl-3,5-heptanedionate) de Cobalt(III), bis(acétylacétonate) de cuivre(II), bis(2,2,6,6- tétraméthyl -3,5-heptanedionate) de cuivre(II), tris(acétylacétonato)gallium (III), acétylacétonate d'hafnium(IV), tris(acétylacétonato)indium(III), tris(acétylacétonate) de fer(III), tris(2,2,6,6- tétraméthyl -3,5-heptanedionate) de fer(III), tris(acétylacétonate) de manganèse(III), bis(acétylacétonate) de nickel(II), bis(acétylacétonate) de palladium(II), bis(trifluoroacétylacétonate) de palladium(II), acétylacétonate de sodium, bis(acétylacétonate) d'oxyde de titane (IV), bis(2,2,6,6-tétraméthyl-3,5-heptanedionate) d'oxyde de titane(IV), tris(2,2,6,6-tétraméthyl-3,5-heptanedionato) de titane(III), tris(2,2,6,6-tétraméthyl-3,5-heptanedionato) oxotitane(IV), dichloro-bis-(2,2,6,6-tétraméthyl-3,5-heptanedionato)titanium(IV), le (diisopropoxyde)bis(acétylacétonate) de titane(IV), di(isopropoxide)bis(2,2,6,6-tétraméthyl-3,5-heptanedionato)titanium(IV), bis(acétylacétonate) de zinc(II), tetrakis(acétylacétonate)zirconium(IV), tetrakis(hexafluoroacétylacétonate) de zirconium(IV), tetrakis(2,2,6,6-tétraméthyl-3,5-heptanedionato)zirconium(IV) et tetrakis(trifluoroacétylacétonate) de zirconium(IV).

Ces complexes organométalliques sont disponibles dans le commerce ou peuvent être préparés par des méthodes connues de l'homme de l'art par exemple par la réaction entre un sel de métal de transition avec l'acétylacétone en présence d'un base tel que décrite par exemple par R.C. Mehrotra dans "Metal Beta-diketonates and Allied Derivatives" Academic Press, 1978 ou par Fackler, J.P., Jr., "Metal β-Ketoenolate Complexes" in Progress in Inorganic Chemistry, F.A. Cotton, Ed., Interscience: New York, 1966, Vol. 7, p.471.

Le bis(acétylacétonate) de calcium(II), tris(acétylacétonate) de chrome(III), le tris(acétylacétonate) de fer(III), le tris(acétylacétonate) de cobalt(III), le bis(acétylacétonate) de zinc(II), le tris(acétylacétonate) d'aluminum(III), et le bis(acétylacétonate) de nickel(II), représentent les complexes organométalliques convenant particulièrement bien pour la mise en oeuvre du procédé selon l'invention, en particulier en raison de leur faible coût.

On peut bien entendu mettre en oeuvre des mélanges de plusieurs complexes organométalliques.

La quantité de complexe organométallique mise en oeuvre peut varier dans de larges limites. Elle est généralement telle que le rapport molaire complexe organométallique/composé **A** est compris entre 0,001 et 0,05, de préférence entre 0,002 et 0,01.

Selon une variante particulièrement préférée de l'invention, le complexe organométallique est associé à un co-catalyseur auquel. La Demanderesse a en effet trouvé que la présence d'un tel co-catalyseur permettait d'accélérer plus encore la formation de composés comprenant au moins un motif β-hydroxy-uréthane et/ou au moins un motif γ-hydroxy-uréthane. Un tel co-catalyseur peut être de préférence choisi dans le groupe comprenant des bases de Lewis, des sels de tetraalkylammonium et leur mélange.

A titre d'exemples non limitatifs de telles base de Lewis, on peut citer les trialkylamines, telles que la triéthylamine, la tributylamine, ou 1,4-diazabicyclooctane; les sels de tetralakyl ammoniums, tels que le bromure de tetrabutylammonium ; les amines hétérocycliques aromatiques telles que la pyridine, la 4-diméthylaminopyridine, ou le N-méthyl imidazole; les trialkylphosphines telles que la triéthylphosphine ou la butylphosphine; les triarylphosphines telles que la triphénylphosphine; les trialkylphosphites telles que la triméthylphosphite, triéthylphosphite, triisopropylphosphite, tri-n-butylphosphite ; ou les triarylphosphites telles que la triphénylphosphite. Parmi les base de Lewis, on préfère tout particulièrement la triéthylamine, la tributylamine ou la 1,4-diazabicyclooctane.

Comme exemple de sels d'ammoniums quaternaires, on peut citer le bromure de tetrabutylammonium et le chlorure de tetrabutylammonium.

On peut bien entendu mettre en oeuvre des mélanges de plusieurs co-catalyseurs.

Le co-catalyseur est généralement utilisé à raison de 0,5 à 3 moles par mole de complexe organométallique, de préférence de 1,5 à 3 moles par mole de complexe organométallique, préférentiellement à raison de 2 moles par mole de complexe organométallique.

Selon un mode de réalisation préféré, le procédé selon l'invention est particulièrement adapté pour la préparation d'un composé comprenant au moins un motif β-hydroxy-uréthane et/ou au moins un motif γ-hydroxy-uréthane à partir d'un composé A comprenant au moins un motif réactif cyclocarbonate et d'un composé B comprenant au moins un motif réactif amino ($-NH_2$).

Un composé A convenant à la mise en oeuvre du procédé de l'invention peut être choisi dans le groupe des composés, oligomères ou polymères terminés par au moins un motif réactif cyclocarbonate, de préférence au moins deux motifs réactifs cyclocarbonates, ces oligomères ou polymères peuvent être linéaires ou ramifiés.

Ainsi, selon une première variante, le composé **A** est un composé répondant à la formule générale **A1 :**

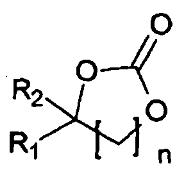

**A1**

dans laquelle :

- n représente un nombre entier égal à 1 ou 2,
- $\mathbf{R_1}$ représente un atome d'hydrogène ou un groupe alkyle en $C_{1-6}$, linéaire ou ramifié, saturé ou insaturé, et le cas

échéant, substitué par un ou plusieurs substituants choisis parmi halogène, hydroxy, cyano, carboxy, trifluorométhyl, alkyle en $C_{1-6}$, alkylamino en $C_{1-6}$, dialkylamino en $C_{1-6}$, alcoxy en $C_{1-6}$, alkylcarboxylate en $C_{1-6}$,

- **$R_2$** représente un atome d'hydrogène ou un groupe choisi parmi : un alkyle en $C_{1-20}$, linéaire ou ramifié, saturé ou insaturé, et dans lequel des unités méthylènes non voisines peuvent être remplacées par des radicaux -O-, -S-,-S(O)-, -$SO_2$-, -O-C(=O)-, -N($R^{21}$)-, -N($R^{21}$)-C(=O)-, -N($R^{21}$)-C(=O)-O-, -N($R^{21}$)-C(=O)-N($R^{22}$)-, avec $R^{21}$ et $R^{22}$ identiques ou différents, représentant des atomes d'hydrogène ou des radicaux choisis parmi alkyle en $C_{1-6}$ ou aryle en $C_{6-14}$; un cycloalkyle en $C_{3-10}$; un aryle en $C_{6-14}$; un hétérocyle en $C_{3-10}$ portant un ou plusieurs hétéroatomes choisis parmi N, O, S; un hétéroaryl en $C_{4-13}$ portant un ou plusieurs hétéroatomes choisis parmi N, O, S; les membres de ce groupe étant éventuellement substitués par un ou plusieurs substituants choisis parmi halogène, hydroxy, cyano, carboxy, trifluorométhyl, alkyl en $C_{1-6}$, alkylamino en $C_{1-6}$, dialkylamino en $C_{1-6}$, alcoxy en $C_{1-6}$, alkylcarboxylate en $C_{1-6}$.

A titre d'exemple non limitatif de composés **A1** utilisables dans le procédé de l'invention, on peut citer le 1,3-dioxolan-2-one; le 4-méthyl-1,3-dioxolan-2-one; le 4-éthyl-1,3-dioxolan-2-one ; le 4-(trifluorométhyl)-1,3-dioxolan-2-one; le diméthyl-1,3-dioxolan-2-one; le 4-éthényl-1,3-dioxolan-2-one; le 4-phényl-1,3-dioxolan-2-one; le 4-méthyl-1,3-dioxan-2-one; le 1,3-dioxan-2-one ; le 4-hydroxyméthyl-1,3-dioxolan-2-one; l'acétate de (2-oxo-1,3-dioxolan-4-yl)méthyl; le 4-(méthoxyméthyl)-1,3-dioxolan-2-one; le 4-(propan-2-yloxy)-1,3-dioxolan-2-one ; le 4-(butoxyméthyl)-1,3-dioxolan-2-one; le 4-(naphthalen-1-yloxyméthyl)-1,3-dioxolan-2-one ; le 4-(phénylméthoxyméthyl)-1,3-dioxolan-2-one; le 4-(hexadécoxyméthyl)-1,3-dioxolan-2-one; le 4-[(E)-octadéc-9-ènoxy]-1,3-dioxolan-2-one ; le 4-(octoxyméthyl)-1,3-dioxolan-2-one ; le 4-(1-hydroxy-2-phényléthyl)-1,3-dioxolan-2-one; le 4-(1-hydroxy-2-phénylméthoxyéthyl)-1,3-dioxolan-2-one; le 4-[[4-(6-méthoxy-2-phényl-3,4-dihydronaphthalen-1-yl)phénoxy]méthyl]-1,3-dioxolan-2-one; le 4-(1-hydroxy-2-phénylméthoxyéthyl)-1,3-dioxolan-2-one.

Selon une autre variante, le composé **A** est un composé répondant à la formule générale **A2 :**

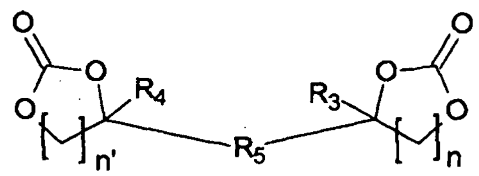

**A2**

dans laquelle :

- n, n', identiques ou différents représentent un nombre entier égal à 1 ou 2,

- **$R_3$** et **$R_4$,** identiques ou différents, représentent un atome d'hydrogène ou un groupe alkyle en $C_{1-6}$, linéaire ou ramifié, saturé ou insaturé, et le cas échéant, substitués par un ou plusieurs substituants choisis parmi halogène, hydroxy, cyano, carboxy, trifluorométhyl, alkyl en $C_{1-6}$, alkylamino en $C_{1-6}$, dialkylamino en $C_{1-6}$, alcoxy en $C_{1-6}$, alkylcarboxylate en $C_{1-6}$,

- **$R_5$** représente :

  - une liaison directe,

  - un groupe bivalent choisi parmi : : un alkylène en $C_{1-20}$, linéaire ou ramifié, saturé ou insaturé, et dans lequel des unités méthylènes non voisines peuvent être remplacées par des radicaux -O-, -S-, -S(O)- -$SO_2$-, -C(=O)-O-,-N($R^{21}$)-, -N($R^{21}$)-C(=O)-, -N($R^{21}$)-C(=O)-O-, -N($R^{21}$)-C(=O)-N($R^{22}$)-, avec $R^{21}$ et $R^{22}$ identiques ou différents, représentant des atomes d'hydrogène ou des radicaux choisis parmi alkyl en $C_{1-6}$ ou aryle en $C_{6-14}$; un cycloalkylène en $C_{3-10}$; un arylène en $C_{6-14}$; un hétérocyclène en $C_{3-10}$ portant un ou plusieurs hétéroatomes choisis parmi N, O, S; un hétéroarylène $C_{4-13}$ portant un ou plusieurs hétéroatomes choisis parmi N, O, S; les membres de ce groupe étant éventuellement substitués par un ou plusieurs substituants choisis parmi halogène, hydroxy, cyano, carboxy, trifluorométhyl, alkyl en $C_{1-6}$, alkylamino en $C_{1-6}$, dialkylamino en $C_{1-6}$, alcoxy en $C_{1-6}$, alkylcarboxylate en $C_{1-6}$,

  - ou, un groupe bivalent présentant la formule générale I suivant:

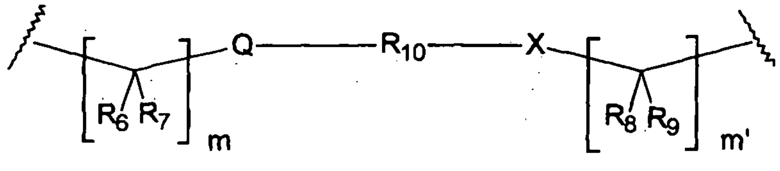

(I)

dans laquelle :

- m et m' identiques ou différents désignent un nombre entier compris entre 1 à 100,

- $R_6$, $R_7$, Re et $R_9$, identiques ou différents, représentent des atomes d'hydrogène ou des radicaux alkyles en $C_{1-6}$, linéaires ou ramifiés, saturés ou insaturés, et le cas échéant, substitués par un ou plusieurs substituants choisis parmi halogène, hydroxy, cyano, carboxy, trifluorométhyl, alkyl en $C_{1-6}$, alkylamino en $C_{1-6}$, dialkylamino en $C_{1-6}$, alcoxy en $C_{1-6}$, alkylcarboxylate en $C_{1-6}$,

- Q et X identiques ou différents représentent un groupe -O-C(=O)-, - $N(R^{21})$-, $-N(R^{21})-C(=O)-$, $-N(R^{21})-C(=O)-O-$, $-N(R^{21})-C(=O)-N(R^{22})-$, avec $R^{21}$ et $R^{22}$ sont tels que définis précédemment,

- $R_{10}$ représente un groupe bivalent choisi parmi : un alkylène en $C_{1-20}$, linéaire ou ramifié, saturé ou non, et dans lequel des unités méthylènes non voisines peuvent être remplacées par des radicaux -O-, -S-, -S(O)- $-SO_2-$,-C(=O)-O-, $-N(R^{21})-$, $-N(R^{21})-C(=O)-$, $-N(R^{21})-C(=O)-O-$, $-N(R^{21})-C(=O)-N(R^{22})-$, avec $R^{21}$ et $R^{22}$ identiques ou différents, représentant des atomes d'hydrogène ou des radicaux choisis parmi alkyl en $C_{1-6}$ ou aryle en $C_{6-14}$; un cycloalkylène en $C_{1-10}$; un arylène en $C_{6-14}$; un hétérocyclène en $C_{3-10}$ portant un ou plusieurs hétéroatomes choisis parmi N, O, S; un hétéroarylène $C_{4-13}$ portant un ou plusieurs hétéroatomes choisis parmi N, O, S; les membres de ce groupe bivalent étant éventuellement substitués par un ou plusieurs substituants choisis parmi halogène, hydroxy, cyano, carboxy, trifluorométhyl, alkyl en $C_{1-6}$, alkylamino en $C_{1-6}$, dialkylamino en $C_{1-6}$, alcoxy en $C_{1-6}$, alkylcarboxylate en $C_{1-6}$;

- ou, un groupe répondant à l'une des formules II ou III :

$$-(CH_2-CH_2-O)_tCH_2-CH_2- \qquad (II),$$

ou

$$-C(=O)-CH_2-O-(CH_2-CH_2-O)_{t'}-CH_2-C(=O)- \qquad (III)$$

dans lesquelles :

- **t, t'**, identiques ou différents, désignent un nombre entier compris entre 1 et 100.

A titre d'exemple non limitatif de composés répondant à la formule A2 on peut citer le 4-(2-oxo-1,3-dioxolan-4-yl)-1,3-dioxolan-2-one ; le bis[(2-oxo-1,3-dioxolan-4-yl)méthyl] hexanedioate et le 2,5-dithiahexane-1,6-diyl-4,4'-bis(1,3-dioxolan-2-one).

Selon encore une autre variante, le composé A est un composé A3 susceptible d'être obtenu par la réaction de condensation du 4-hydroxyméthyl-1,3-dioxolan-2-one avec un composé comprenant deux ou plusieurs fonctions isocyanates, tel que le méthylène diphényl diisocyanate ; le 1,3-diisocyanatobenzène; le 1,4-diisocyanatobenzène ; le 2,4-diisocyanato-1-méthylbenzène ; le 1-isocyanato-4-[(4-isocyanatocyclohexyl)méthyl]cyclohexane ; le 1,5-diisocyanatonaphthalène ; 1,3-diisocyanato-2-méthylbenzène ; le 1,6-diisocyanatohexane ; le 1-isocyanato-4-(4-isocyanato-3-méthylphényl)-2-méthylbenzène ; le 1-isocyanato-4-(4-isocyanato-3-méthoxyphényl)-2-méthoxybenzène ; le 1,3-bis(isocyanatométhyl)benzène; 1-isocyanato-4-(isocyanatométhyl)benzène ; le 5-isocyanato-1-(isocyanatométhyl)-1,3,3-triméthylcyclohexane ; le 1,4-diisocyanatocyclohexane ; le 2-[2-[[3-(isocyanatométhyl)phényl]carbamoyloxy]éthoxy]éthyl N-[3-(isocyanatométhyl)phényl]carbamate ; le 1,3-diisocyanato-5-[(2-isocyanatophényl)méthyl]-2-méthylbenzène ; 1,3-diisocyanato-S-[(2-isocyanatophényl)méthyl]-2-méthylbenzène ; le 1,10-diisocyanatodécane; le 1,6-diisocyanato-2,3,4-triméthylhexane ; le 1,5-diisocyanato-2,2-diméthylpentane ; le 1,8-diisocyanatooctane ; le 1,4-diisocyanatobutane ; le 1,4-bis(isocyanatométhyl)cyclohexane ; le 3-[[3-(isocyanatométhyl)phényl]carbamoyloxy]butyl N-[3-(isocyanatométhyl)phényl]carbamate ; le 1,3-bis[(5-isocyanato-1,3,3-triméthylcyclohexyl)méthyl]urée ; le 1,6,11-undécane triisocyanate ; l'ester éthyl L-lysine triisocyanate ; le triphényl méthane triisocyanate) ; le 1,3-bis(6-isocyanatohexyl)-1-(6-isocyanatohexylcarbamoyl)urea ; le 1,3-diisocyanato-5-[(4-isocyanatophényl)méthyl]-2-méthylbenzène ; le méthyl 6-[2,2-bis[(5-isocyanato-6-méthoxy-6-oxohexyl)carbamoyloxyméthyl] butoxycarbonylamino]-2-isocyanatohexanoate, ou leurs mélanges.

Le composé **A3** peut également être obtenu par la réaction d'estérification du 4-hydroxyméthyl-1,3-dioxolan-2-one avec un composé comprenant deux ou plusieurs fonctions acides carboxyliques tels que les diacides aliphatiques ou les diacides aromatiques. Comme exemples de diacides aliphatiques on peut citer l'acide éthanedioïque;- l'acide 1,3-propanedioïque ; l'acide 1,4-butanedioïque ; l'acide 1,5-pentanedioïque ; l'acide 1,6-hexanedioïque ; l'acide 1,7-heptanedioïque ; l'acide 1,8-octanedioïque ; l'acide 1,9-nonanedioïque ; l'acide 1,10-décanedioïque ; l'acide 1,11-undécanedioïque ou l'acide 1,12-dodécanedioïque. Comme exemples de diacides aromatiques on peut citer l'acide benzène-1,2-dicarboxylique ; l'acide benzène-1,3-dicarboxylique ou l'acide benzène-1,4-dicarboxylique.

Le composé **A3** peut aussi être obtenu par la réaction de condensation du triglycidyl isocyanurate (polyscience Inc.) avec le dioxyde de carbone.

Le composé **A3** peut aussi être obtenu par la réaction d'un composé comprenant deux ou plusieurs groupes éthers de glycidyle avec le dioxyde de carbone en présence d'un catalyseur telle que décrite dans les documents de brevet US5340889 (Crawford et al.), US7232877 (Figovsky et al.), US5175231 (Rappoport et al.), US6495637 (Rappoport et al.). A titre d'exemples de composés comprenant deux ou plusieurs groupes éthers de glycidyle, on peut citer, le 2-[2-(oxiran-2-ylméthoxy)éthoxyméthyl]oxirane (Quetol 651 ; Polyscience Inc.); 2-[3-(oxiran-2-yiméthoxy)propoxyméthyl]oxirane (polyscience Inc.), le 2-[4-(oxiran-2-ylméthoxy)butoxyméthyl]oxirane (polyscience Inc.) ; le 2-[2,2-bis(oxiran-2-ylméthoxyméthyl)butoxyméthyl]oxirane (trimethylolpropane triglycidyl ether); le 2-[[2-méthyl-3-(oxiran-2-ylméthoxy)-2-(oxiran-2-ylméthoxyméthyl)propoxy]méthyl]oxirane ; le 2-[[3-(oxiran-2-ylméthoxy)-1-(oxiran-2-ylméthoxyméthyl)propoxy]méthyl]oxirane ; le 2-[[3-(oxiran-2-ylméthoxy)-2,2-bis(oxiran-2-ylméthoxyméthyl)propoxy]méthyl]oxirane (ou Pentaérythritol glycidyl éther) ; N,N-diglycidyl-4-glycidyloxyaniline (Aldrich®); un di- ou polyglycidyle éther de la série HELOXY® (HEXION® specialty chemicals) ; le bis(polyoxyéthylène bis[glycidyl éther]) (Sigma-Aldrich Co.) ; le poly(propylène glycol) (600) bis[glycidyl éther] (polyscience Inc.); ou une résine époxy choisie parmi celles décrites dans le brevet US6410127 (Toray Industries, Inc) ou encore une résine époxy bisphénol choisie parmi celles commercialisées par la société Dow (résine époxy bisphénol A; résine époxy bisphénol F; résine époxy bisphénol A/F; résine époxy bisphénol A modifiée; résine époxy bisphénol A/F modifiée).

On peut bien entendu mettre en oeuvre des mélanges de composés comprenant deux ou plusieurs motifs réactifs cyclocarbonates, par exemple pour la préparation de polyuréthanes hydroxylés.

Le composé **B** utile dans le procédé de l'invention peut être choisi dans le groupe des composés, oligomères ou polymères terminés par au moins un motif réactif ($-NH_2$), de préférence par au moins deux motifs réactifs ($-NH_2$), ces oligomères ou polymères pouvant être linéaires ou ramifiés.

Avantageusement, le ou les motifs réactifs ($-NH_2$) sont portés par des carbones saturés (sp3), lesquels sont également porteurs d'au moins un hydrogène, de préférence de deux hydrogènes. A titres d'exemple de composé B convenant à la mise en oeuvre du procédé selon l'invention, on peut citer : butylamine ; hexylamine ; cyclohexylamine ; éthanolamine ; propanolamine ; éthylène diamine ; propylène diamine (ou 1,4-diaminopropane ou triméthyléne diamine); butylène diamine (1,4-diaminobutane ou tetraméthylène diamine); pentaméthylène diamine ; hexaméthylène diamine ; heptaméthylène diamine ; tétraméthylène diamine ; octaméthylène diamine ; nonaméthylène diamine ; décaméthylène diamine ; méthyl-2-pentaméthylène diamine ; undécaméthylène diamine ; dodécaméthylène diamine ; xylylène diamine : isophorone diamine ; triméthyl hexaméthylène diamine (2,2,4-triméthyl hexaméthylène diamine et/ou 2,4,4-triméthyl hexaméthylène diamine ou VESTAMIN® TMD); 1,2-diaminocyclohexane ; 1,4-diaminocyclohexane ; 4,4'-diaminocyclohexyl méthane (1,4-bis(aminocyclohexyl)méthane); 2-(2-aminoéthoxy)éthanol ; bis-(3-méthyl4-aminocyclohexyl)méthane ; 2,2-bis-(4-aminocyclohexyl)propane ; nitrile tris(éthaneamine) ; bis-(3-aminopropyl)méthylamine ; 3-amino-1-(méthylamino)propane ; 3-amino-1-(cyclohexylamino)propane ; N-(2-hydroxyéthyl)éthylène diamine; 4,7-dioxadécane-1,10-diamine ; 4,9-dioxadodécane-1,12-diamine; 7-méthyl4,10-dioxatridécane-1,1 3-diamine; une polyéther monoamine de préférence choisie parmi l'hydroxy polyéthyléne glycol amine et la méthoxypolyéthyléne glycol amine de la série des JEFFAMINE® M (exemple M-600 (XTJ-505), M-1000 (XTJ-506), M-2005, M-2070); une polyéther diamine, de préférence choisie parmi une polyoxyéthylène diamine et une polyoxypropylène diamine de la série des JEFFAMINE® D, ED et/ou EDR (exemple JEFFAMINE® D-230 , D-400 ou D-2000 ; EDR-148 (XTJ-504) EDR-176 (XTJ-590) ; HK-511 ; ED-600 (XTJ-500) ; ED-900 (XTJ-501) ; ED-2003 (XTJ-502).

Le composé **B** utilisable dans le procédé selon l'invention peut aussi être choisi parmi les triamines, tétramines, les polyoxyéthylènes triamines choisies dans la série des JEFFAMINE® T (exemple T-403 ; T-3000 (XTJ-509) ; T-5000) et les oligomères ou polymères terminés par au moins un motif réactif amino ($-NH_2$), de préférence par deux ou plusieurs motifs réactifs amino ($NH_2$), lesdits oligomères ou polymères pouvant avoir un squelette polyamide, polyéther, et/ou polyéster. (exemple : un polyamido polyamine tels que ceux préparés par le procédé décrit dans le brevet US5391826

(Speranza et al.)].

En général, le composé A comprenant au moins un motif réactif cyclocarbonate et le composé B comprenant au moins un motif réactif amino (-$NH_2$) sont mélangés de manière à ce que le rapport molaire composé B sur composé A soit compris entre 0,5 et 2, de préférence égal à 1. Le rapport molaire pouvant varier autour du rapport stoechiométrique suivant la nature souhaitée du groupe terminal dans le produit final.

La présence d'un solvant n'est pas nécessaire pour la réalisation du procédé selon l'invention. Toutefois dans certaines circonstances, la présence du solvant peut être souhaitée en particulier pour solubiliser le complexe organométallique et/ou pour homogénéiser le milieu réactionnel. La quantité de solvant à utiliser dans de cas n'est pas critique.

Parmi les solvants convenant à la mise en oeuvre du procédé selon l'invention on peut citer l'eau; les alcools tels que le méthanol, l'éthanol, le 1-propanol, le 2-propanol, le 2-méthyl-1-propanol, le 1-butanol, le 2-butanol, , tert-butanol, le 1-pentanol, le 2-pentanol, le 3-pentanol, le 1-hexanol, le 2-méthyl-1-pentanol, le 2-éthyl-1-butanol, le 1-heptanol, le 1-octanol, le 2-octanol, le 1-nonanol or le 1-décanol; les hydrocarbures aliphatiques ou aromatiques, halogénés ou non, tels que le n-hexane, le n-heptane, l'isooctane, le nonane, le décane, le chlorure de méthylène, le chloroforme, le benzène, le chlorobenzène, le toluène, le o-xylène, le m-xylèrie, le p-xylène, l'éthylbenzène, le cyclohexane ou le méthylcyclohexane ; Les éthers tels que le tetrahydrofurane, le dioxane, le tetrahydropyrane, le 1,2-diméthoxyéthane, le 1,2-diéthoxyéthane, le diéthylène glycol diméthyl éther ou le diéthylène glycol diéthyl éther; les cétones tels que l'acétone ou la méthyl éthyl cétone; les sulfoxydes tels que diméthylsulfoxyde ; les amides tels que la diméthylformamide ou la N-méthyl pyrrolidone ; les nitriles tels que l'acétonitrile ; ou les amines aromatiques telles que la pyridine, Les solvants convenant particulièrement bien pour la mise en oeuvre du procédé selon l'invention sont choisis parmi les solvants polaires présentant un bas point d'ébullition comme le tetrahydrofurane, l'acétone, ou la méthyléthylcétone. Il a en effet été démontré que ce type de solvant permettait notamment d'homogénéiser le milieu réactionnel et de favoriser la réaction de condensation.

On peut bien entendu mettre en oeuvre des mélanges de plusieurs solvants.

Grâce à la combinaison d'un catalyseur organométallique et d'un co-catalyseur, la présente invention offre la possibilité de préparer des composés comprenant au moins un motif β-hydroxy-uréthane et/ou au moins un motif γ-hydroxy-uréthane dans des conditions de de temps et de températures acceptables sur le plan industriel, de préférence à des températures inférieures à 50°C, préférentiellement à la température ambiante (environ 25°C).

Bien qu'il soit possible de mettre en oeuvre le procédé de l'invention à une pression supérieure à la pression atmosphérique, on préfère le plus souvent opérer à la pression atmosphérique.

La durée de la réaction de condensation dépend de la température et de la nature du composé **A** à condenser avec le composé B ainsi que du catalyseur mis en oeuvre. Elle peut varier entre 0.01 et 600 minutes, en particulier, la durée de réaction est supérieure à 1 minute et ne dépasse pas 480 minutes. Un suivi de l'état d'avancement de la réaction par prélèvement et évaluation du produit formé par la technique de spectroscopie Infrarouge à Transformée de Fourier (FTIR) permet à l'homme de l'art de déterminer aisément la durée de réaction la plus appropriée aux conditions utilisées.

Le procédé selon l'invention peut être conduit sous agitation mécanique ou magnétique. Il peut être mis en oeuvre dans n'importe quel réacteur ou appareillage permettant de réunir les conditions décrites ci-avant.

Le procédé selon l'invention peut être mené aussi bien en continu qu'en discontinu. Industriellement, on préfère le procédé en continu.

Le composé **A** et le composé B peuvent être mis en contact avec le catalyseur comprenant le complexe organométallique et le cas échéant le co-catalyseur, de diverses manières connues en soi.

Un mode de réalisation particulièrement préféré de l'invention consiste à réaliser au préalable le mélange du composé A et du composé B, puis à introduire successivement le complexe organométallique et le co-catalyseur dans le mélange sous agitation mécanique, à la température ambiante et à la pression atmosphérique, et enfin à ajouter le solvant pour homogénéiser le mélange réactionnel ainsi obtenu.

A la fin de la réaction, le milieu réactionnel peut être soumis à diverses techniques de séparation ou de purification connues telles que l'évaporation du solvant (et le cas échéant du co-catalyseur) et séchage sous vide.

Les rendements bruts élevés obtenus dans ces conditions facilitent l'obtention de composés comprenant au moins un motif β-hydroxy-uréthane et/ou au moins un motif γ-hydroxy-uréthane de très grande qualité.

La présente invention concerne également l'utilisation d'un catalyseur comprenant au moins un complexe organométallique et un co-catalyseur choisi dans le groupe des bases de Lewis et/ou des sels de tétra-alkyl ammonium, pour catalyser la réaction de condensation entre un composé A comprenant au moins un motif réactif cyclocarbonate et un composé B comprenant au moins un motif réactif amino (-$NH_2$). Le complexe organométallique mis en oeuvre dans une telle utilisation contient un métal alcalin, alcalino-terreux ou de transition choisi parmi les groupes IA, IIA, IIIA, IIIB, IVA, IVB, VB, VIB, VIIB et VIII du tableau périodique, et au moins deux ligands acétylacétonato.

Le procédé de la présente invention permet donc la préparation de composés comprenant au moins un motif β-hydroxy-uréthane et/ou au moins un motif γ-hydroxy-uréthane de haute qualité, ne présentant pas les inconvénients liés aux uréthanes préparés selon les modes opératoires connus de l'art antérieur, c'est-à-dire l'aspect collant des

résines d'hydroxyuréthanes préparés à partir de cyclocarbonates par des procédés non quantitatifs ou les troubles causés par les isocyanates résiduels dans les matériaux à base de polyuréthanes conventionnels.

Ainsi, le procédé de la présente invention permet d'envisager une utilisation plus facile, plus sûre, et donc plus importante, des hydroxyuréthanes, en particulier des composés comprenant au moins un motif β-hydroxy-uréthane et/ou au moins un motif γ-hydroxy-uréthane, notamment dans les domaines de revêtements, résines de coulées, vernis souples, résines d'imprégnation pour l'isolation électrique ou autres, mais aussi dans les domaines cosmétique, pharmaceutique ou biomédical ou encore comme intermédiaires pour la préparation de résines réticulées.

Dans le cadre de la préparation d'hydroxyuréthanes (oligomères linéaires ou ramifiés ou polymères linéaires ou ramifiés) selon le procédé de la présente invention, le mélange formé à partir du composé **A** et du composé B peut en outre comprendre un plastifiant (inerte) avant la mise en contact avec le catalyseur comprenant le complexe organométallique et le cas échéant le co-catalyseur. Le plastifiant (inerte) ayant pour fonction d'abaisser la viscosité du mélange composé **A**/composé **B,** ainsi que celle du produit hydroxyuréthane final, en vue d'améliorer la processabilité de ce dernier, en particulier de faciliter l'application automatisée en coulée. A titre d'exemple de plastifiants pouvant convenir pour la préparation d'hydroxyuréthanes (oligomères linéaires ou ramifiés ou polymères linéaires ou ramifiés) selon le procédé de la présente invention on peut citer les plastifiants décrits dans le brevet US5908894 (Genz et al.). De préférence, le plastifiant est choisi parmi les esters d'acide phtalique tels que le phtalate de benzylbutyle (BBP), le phtalate de dibutyle (DBP), le phtalate de diéthyle(DEP), le phthalate de dioctyle (DOP) ou, le phtalate de di-2-éthylhexyle (DEHP) ; les esters d'acide succinique ; les esters d'acide adipique tels que l'adipate de dibutyle ou l'adipate de dioctyle ; les esters d'acide citrique tels que le 2-(acétyloxy)-1,2,3-propanetricarboxylate de tributyle; les esters d'isosorbide tels que le Polysorb® ID 37 EXP commercialisé par l'entreprise Roquette Frères® S.A.; les sulfonamides tel que le toluène sulfonamide commercialisé par l'entreprise Axcentive® S.A.; les esters de phosphates ou les esters d'acide alkylsulfonique. Préférentiellement encore, le plastifiant est choisi parmi les plastifiants issus de ressources renouvelables tels que les di-esters d'isosorbide.

La quantité de plastifiant mise en oeuvre peut varier de 1 à 1000 moles de plastifiant par mole de composé **A.**

Dans un mode de réalisation préféré, le plastifiant est introduit dans le réacteur avant l'introduction du composé **A** et du composé **B.**

A partir de la description qui vient d'être faite de multiples variantes de réalisation du procédé de l'invention peuvent être conçues par l'homme du métier sans sortir du cadre de l'invention définie par les revendications.

La présente invention est également décrite à l'aide des exemples ci-après, donnés à titre d'illustration sans toutefois en limiter la portée.

## Exemples

### Exemple 1: Etude comparative de l'activité catalytique de catalyseurs mis en oeuvre dans la réaction entre un composé cyclocarbonate et une diamine.

Dans cet exemple, l'activité catalytique du catalyseur comprenant le tris(2,4-pentanedione)chrome(III) (SACHEM Europe BV), et éventuellement associé à la triéthylamine a été comparée aux catalyseurs préconisés dans les méthodes connues de l'art antérieur, à savoir Bétaine (Sigma), Hydroxyde de tetraéthylammonium (Aldrich), 2-hydroxypyridine (Aldrich), tetraéthylammonium tetrafluoroborate (Aldrich), bromure de tetraméthylammonium (Fluka), triphénylphosphine (Aldrich), triéthylamine (Sigma Aldrich), carbonate de calcium (Aldrich). Cette étude a été réalisée sur la réaction de condensation entre la triméthylhexaméthylène diamine (VESTAMIN TMD, Evonik) et le 4-(hydroxyméthyl)-1,3-dioxolan-2-one (Hunstman). La série d'expériences réalisée est explicitée dans le Tableau 1.

Tableau 1: Conditions expérimentales de l'étude des activités catalytiques des différents catalyseurs testés de la réaction de condensation entre la triméthylhexaméthylène diamine et le 4-(hydroxyméthyl)-1,3-dioxolan-2-one.

| Quantité utilisée en 4-(hydroxyméthyl)-1,3-dioxolan-2-one | Quantité utilisée en Triméthylhexaméthylène diamine | Nature du catalyseur [quantité utilisée] |
|---|---|---|
| 3,00 g (25,4 mmol) | 4,01 g (25,4 mmol) | Absence |
| 3,00 g (25,4 mmol) | 4,01 g (25,4 mmol) | Bétaine [3,84 mg ; 0,19 mmol] |
| 3,00 g (25,4 mmol) | 4,01 g (25,4 mmol) | Hydroxyde de tétraéthylammonium [27,90 mg ; 0,19 mmol] |
| 3,00 g (25,4 mmol) | 4,01 g (25,4 mmol) | 2-Hydroxypyridine [18,00 mg ; 0,19 mmol] |

(suite)

| Quantité utilisée en 4-(hydroxyméthyl)-1,3-dioxolan-2-one | Quantité utilisée en Triméthylhexaméthylène diamine | Nature du catalyseur [quantité utilisée] |
|---|---|---|
| 3,00 g (25,4 mmol) | 4,01 g (25,4 mmol) | Tetraéthylammonium tetrafluoroborate [41,20 mg ; 0,19 mmol] |
| 3,00 g (25,4 mmol) | 4,01 g (25,4 mmol) | Carbonate de calcium [19,00 mg ; 0,19 mmol] |
| 3,00 g (25,4 mmol) | 4,01 g (25,4 mmol) | Bromure de tetraméthylammonium [29,20 mg ; 0.19mmol] |
| 3,00 g (25,4 mmol) | 4,01 g (25,4 mmol) | Triphénylphosphine [49,80 mg ; 0,19 mmol] |
| 3,00 g (25,4 mmol) | 4,01 g (25,4 mmol) | Triéthylamine [19,20 mg ; 0,19 mmol] |
| 3,00 g (25,4 mmol) | 4,01 g (25,4 mmol) | Tris(2,4-pentanedione) chrome III [40,53 mg ; 0,12 mmol] |
| 3,00 g (25,4 mmol) | 4,01 g (25,4 mmol) | Tris(2,4-pentanedione)chrome III [12,80 mg ; 0,04 mmol] et Triéthylamine [3,84 mg ; 0,04 mmol] |

La conversion du 4-(hydroxyméthyl)-1,3-dioxolan-2-one en l'hydroxyuréthane correspondant a été suivie par spectroscopie FTIR avec un appareil Nicolet 510P (Figure 1).

Afin, de s'affranchir des variations des intensités des bandes entre les différentes analyses réalisées, une approximation de la conversion ($\alpha$) de ces fonctions a été définie à partir du rapport d'aire (r). Celui-ci correspond au ratio entre l'aire de la bande caractéristique du groupe carbonyle appartenant à l'hétérocycle 1,3-dioxolan-2-one située à 1800 $cm^{-1}$ et de celle centrée à 769 $cm^{-1}$ attribuée au $C_{sp2}$-H de l'hétérocycle oxygéné (Figure 1) :

$$r(t) = \frac{Aire\ \nu_{C=O}(t)}{Aire\ \nu_{Csp2-H}(t)}$$

On en déduit la conversion en 4-(hydroxyméthyl)-1,3-dioxolan-2-one $\alpha$(t) au cours du temps :

$$\alpha(t) = \frac{r(0) - r(t)}{r(0)}$$

Les résultats obtenus sont présentés à la Figure 2 dans laquelle les chiffres entre crochets correspondent aux catalyseurs utilisés : [1] Bétaine, [2] tetraéthylammonium tetrafluoroborate, [3] hydroxyde de tetraéthylammonium, [4] 2-hydroxypyridine, [6] carbonate de calcium, [7] triphénylphosphine, [8] bromure de tetraméthylammonium, [9] triéthylamine, [10] le tris(2,4-pentanedione)chrome(III), [11] catalyseur comprenant le tris(2,4-pentanedione)chrome(III) et la triéthylamine.

A partir des données présentées Figure 2, il apparaît que l'utilisation de tris(2,4-pentanedione)chrome(III) éventuellement additivé de triéthylamine permet une accélération de la réaction de condensation entre le 4-(hydroxyméthyl)-1,3-dioxolan-2-one et la diamine très supérieure aux accélérations induites par les emplois des catalyseurs précédements décrits dans les propositions techniques. Ce système catalytique permet d'atteindre une conversion quantitative du réactif cyclocarbonaté dans un intervalle de temps très court de l'ordre de 120 secondes.

<u>**Exemple 2** : **Catalyse par le système tris(2,4-pentanedione)chrome(III) / triéthylamine de l'addition de la triméthylhexaméthylène diamine sur l' acétate d'(2-oxo-1,3-dioxolan-4-yl)méthyle.**</u>

*1. <u>Préparation de l'acétate d'(2-oxo-1,3-dioxolan-4-yl)méthyle</u>*

Dans un ballon bicol de 250 ml surmonté d'un réfrigérant, sont introduits respectivement: le 4-(hydroxyméthyl)-1,3-dioxolan-2-one (29,00 g, 0,22 mol), l'anhydride acétique (22,20 g, 0,21 mol, Sigma, Aldrich) et 2 gouttes d'acide sulfurique pur. Le mélange réactionnel placé sous agitation mécanique est porté à 60°C, sous air et à pression atmosphérique durant 45 minutes.

Le brut réactionnel est repris par 80 ml de chloroforme. La phase organique est lavée une dizaine de fois par 200 ml d'eau distillée puis, séchée sur sulfate de magnésium anhydre.

Le chloroforme est évaporé sous vide. Le produit est concentré à l'évaporateur rotatif sous vide à 40°C. 20 g d'une huile peu visqueuse, de couleur jaune sont obtenus (Rendement = 39,2%).

*RMN$^1$H (DMSO):* δ=2,5 ppm (3H, $C_4H$), 4,65 ppm (1 H, $C_1H$), 4,70 ppm (1H, $C_3H$), 4,77 ppm (1H, $C_3H$), 5,12 ppm (1H, $C_1H$), 5,5 ppm (1H, $C_2H$). Ce spectre RMN $^1$H a été enregistré avec un appareil 400MHz Bruker AC 400.

*2. <u>Addition de la triméthylhexaméthyléne diamine sur l'acétate d'(2-oxo-1,3-dioxolan-4-yl)méthyle en présence de tris(2,4-pentanedione)chrome(III) / triéthylamine</u>*

Dans un tube à hémolyse sont introduits en absence de solvant, l'acétate d'(2-oxo-1,3-dioxolan-4-yl)méthyle (4,06 g, 25,40 mmol), la triméthylhexaméthylène diamine (4,01 g, 25,40 mmol), le tris(2,4-pentanedione)chrome(III) (12,80 mg, 0,04 mmol) et la triéthylamine (3,84 mg, 0,04 mmol).

Le mélange réactionnel placé sous agitation mécanique est laissé à température ambiante, sous air et à pression atmosphérique. La consommation de l'acétate d'(2-oxo-1,3-dioxolan-4-yl)méthyle après le mélange des réactifs a été définie par spectroscopie FTIR (Figure 3) suivant le protocole décrit dans l'exemple 1.

L'étude de la conversion de l'acétate d'(2-oxo-1,3-dioxolan-4-yl)méthyle en l'hydroxyuréthane correspondant, réalisée par spectroscopie infrarouge met en évidence une conversion quantitative en moins de 3 minutes à température ambiante de ce réactif. Par conséquent, l'utilisation du système catalytique constitué de tris(2,4-pentanedione)chrome(III) additivé de triéthylamine demeure très efficace pour accélérer la réaction de condensation entre la triméthylhexaméthylène diamine et l'acétate d'(2-oxo-1,3-dioxolan-4-yl)méthyle.

<u>**Exemple 3: Catalyse par le système tris(2,4-pentanedione)chrome(III)** / **triéthylamine de l'addition de la tétraéthylène pentamine sur** le 4-**(hydroxyméthyl)-1,3-dioxolan-2-one.**</u>

Dans un tube à hémolyse sont introduits en absence de solvant, le 4-(hydroxyméthyl)-1,3-dioxolan-2-one (3,00 g, 25,4 mmol), la tétraéthylène pentamine (5,10 g, 25,4 mmol, Hunstman), le tris(2,4-pentanedione)chrome(III) (12,80 mg, 0,04 mmol) et la triéthylamine (3,84 mg, 0,04 mmol). Le mélange réactionnel placé sous agitation mécanique est laissé à température ambiante, sous air et à pression atmosphérique.

La conversion de la 4-(hydroxyméthyl)-1,3-dioxolan-2-one en hydroxyuréthane correspondant a été suivie par spectroscopie FTIR, suivant le protocole décrit dans l'exemple 1. Les résultats obtenus sont présentés à la Figure 4.

A partir des données présentées Figure 4, il apparait évident que l'introduction en quantités catalytiques de tris(2,4-pentanedione)chrome(III) et de triéthylamine permet d'atteindre une consommation quantitative à température ambiante de la 4-(hydroxyméthyl)-1,3-dioxolan-2-one dans les 60 secondes suivant le mélange de la tétraéthylène pentamine avec la 4-(hydroxyméthyl)-1,3-dioxolan-2-one.

<u>**Exemple 4** : **Catalyse par le système** le **tris(2,4-pentanedione)chrome(III)/triéthylène amine de la réaction de condensation entre la 3-aminométhyl-3,5,5-triméthylcyclohexylamine et la 4-(hydroxyméthyl)-1,3-dioxolan-2-one.**</u>

Dans un tube à hémolyse sont introduits en absence de solvant la 4-(hydroxyméthyl)-1,3-dioxolan-2-one (3 g, 25,4 mmol), la 3-aminométhyl-3,5,5-triméthylcyclohexylamine (4,32 g, 25,4 mmol, Vestamin IPD Evonik), le tris(2,4-pentanedione)chrome(III) (12,8 mg, 0,04 mmol) et la triéthylamine (3,84 mg, 0,04 mmol). Le mélange réactionnel placé sous agitation mécanique est laissé à température ambiante, sous air et à pression atmosphérique. L'étude de l'avancement de la réaction est réalisée par spectroscopie FTIR, suivant le protocole précédemment décrit.

L'étude de la conversion de la 4-(hydroxyméthyl)-1,3-dioxolan-2-one en l'hydroxyuréthane correspondant par spectroscopie FTIR (Figure 5) au cours de la réaction d'addition réalisée à température ambiante, met en exergue que ce système catalytique permet de consommer quantitativement à température ambiante les fonctions réactives dans un intervalle de temps de l'ordre d'une heure suivant le mélange des réactifs.

<u>**Exemple 5** : **Catalyse par le système le tris(2,4 pentanedione)chrome(III)/triéthylamine de l'addition de la Jeffamine D230 sur la 4-(hydroxyméthyl)-1,3-dioxolan-2-one.**</u>

Dans un tube à hémolyse sont introduits en absence de solvant, la 4-(hydroxyméthyl)-1,3-dioxolan-2-one (3,0 g, 25,4 mmol), la Jeffamine D230 (5,84 g, 25,4 mmol, Jeffamine D230 Hunstman), le tris(2,4-pentanedione)chrome(III)

(12,80 mg, 0,04 mmol, SACHEM Europe BV), et la triéthylamine (3,84 mg, 0,04 mmol, Sigma Aldrich). Le mélange réactionnel placé sous agitation mécanique est laissé à température ambiante, sous air et à pression atmosphérique. Le suivi de l'avancement de la réaction est réalisé par spectroscopie FTIR, suivant le protocole précédemment décrit.

A partir des données présentées Figure 6, il est observé que l'introduction en quantités catalytiques de tris(2,4-pentanedione)chrome(III) additivé de triéthylamine permet d'atteindre une consommation quantitative, à température ambiante, du réactif cylocarbonaté dans un intervalle de temps de 2h suivant le mélange de la Jeffamine D230 avec la 4-(hydroxyméthyl)-1,3-dioxolan-2-one.

**Exemple 6** : **Addition de la tetraéthylènepentamine diamine sur un bis-cyclocarbonate catalysée par le système tris(2,4-pentanedione)chrome(III)/triéthylène amine.**

*1. Synthése d'un bis-cyclocarbonate par addition de la 4-(hydroxyméthyl)-1,3-dioxolan-2-one sur le Desmodur N3400*

Dans un ballon bicol de 250mL préalablement séché et taré, sont introduits respectivement le prépolymère diisocyanate sur base hexaméthylène diisocyanate (0,26 moles, 100 g Desmodur N3400 Bayer), la 4-(hydroxyméthyl)-1,3-dioxolan-2-one (0,53 moles, 61,4 g) et le dilaurate de dioctyl étain (0,001 moles, 0,744 g, Thorson Chemical GmbH). Le mélange réactionnel est laissé sous agitation magnétique à température ambiante et sous pression atmosphérique pendant 2h.

Le brut réactionnel est solubilisé dans 100 ml de tetrahydrofuran puis précipité dans 1 L d'eau distillée. Cette procédure est répétée une deuxième fois. Le précipité est dissout dans du tetrahydrofuran (100 mL) puis séché sur sulfate de magnésium anhydre. Le produit est concentré à l'évaporateur rotatif sous vide à 40°C. 150g d'une huile très visqueuse de couleur blanche sont obtenus (Rendement = 93,0%).

L'analyse en spectroscopie FTIR du produit obtenu met en évidence l'absence de la bande centrée à 2250 $cm^{-1}$ caractéristique des fonctions isocyanates (-NCO) du Desmodur N3400 et la présence à 1783 $cm^{-1}$ de la bande du carbonyl (C=O) du cyclocarbonate.

*2. Catalyse par le système tris(2,4-pentanedione)chrome(III)/ triéthylamine de la polyaddition de la tetraéthylènepentamine sur le bis-cydocarbonate dérivé du Desmodur N3400.*

Dans un bécher sont introduits: le bis-cyclocarbonate dérivé du Desmodur N3400 (16,46 g, 25,4 mmol), la tétraéthylène pentamine (5,10 g, 25,4 mmol, Hunstman), le tris(2,4-pentanedione)chrome(III) (12,8 mg, 0,04 mmol, SACHEM Europe BV) et la triéthylamine (3,84 mg, 0,04 mmol, Sigma Aldrich). Le milieu réactionnel est homogénéisé par l'addition de 2,0 ml de tetrahydrofurane.

**Exemple 7: Etude de la catalyse de la réaction de condensation entre la 3-aminométhyl-3,5,5-triméthylcyclohexylamine et le carbonate de glycérol par différents catalyseurs.**

On utilise un mode opératoire similaire à celui des exemples précédents : mêmes conditions de température et de pression.

Les catalyseurs (complexes organométalliques / co-catalyseurs) testés sont les suivants : [11] tris(acétylacétonate) de chrome(III) / triéthylamine; [12] tris(acétylacétonate) d'aluminium(III) / triéthylamine; [13] bis(acétylacétonate) de calcium(II); [14] bis(acétylacétonate) de calcium(II) / bromure de tetrabutylammonium; [15] bis(acétylacétonate) de calcium(II) / triéthylamine; [16] bis(acétylacétonate) de cobalt (II) / triéthylamine; [17] bis(acétylacétonate) de nickel (II) / triéthylamine; [18] tris(acétylacétonate) de fer(III) / triéthylamine; [19] bis(acétylacétonate) de zinc (II) / triéthyalmine.

Les complexes organométalliques suivants tris(acétylacétonate) de chrome(III), tris(acétylacétonate) d'aluminium(III); bis(acétylacétonate) de cobalt (II); bis(acétylacétonate) de nickel (II); tris(acétylacétonate) de fer(III); bis(acétylacétonate) de zinc (II); bis(acétylacétonate) de calcium(II) sont disponibles chez SICCANOR®. Le bromure de tetrabutylammonium est disponible chez Sigma Aldrich®.

Pour chaque essais, on a utilisé : 10 g (58,8 mmol) de la 3-aminométhyl-3,5,5-triméthylcyclohexylamine (isophorone diamine) et 6,9 g (58,4 mmol) de la 4-(hydroxyméthyl)-1,3-dioxolan-2-one et le système catalytique testé « 0,06 mmol de complexe organométallique en combinaison avec 0,06 mmol le co-catalyseur ».

Ces essais ont montré que les différents catalyseurs utilisés permettent des temps de réaction inférieurs à 5 minutes à température ambiante. Les meilleurs résultats ont été obtenus avec le complexe organométallique bis(acétylacétonate) de calcium(II) en combinaison avec le co-catalyseur triéthylamine. En effet, il a été constaté un temps de réaction inférieur à 30 secondes avec le catalyseur bis(acétylacétonate) de calcium(II) (0,06 mmol) / triéthyalmine(0,12 mmol) à température ambiante.

**Exemple 8 : réaction de condensation entre la 2,2,4-triméthylhexaméthylène diamine et un mélange composé d'un tricyclocarbonate (Laprolat ETF) et d'un dicyclocarbonate (Laprolat E-181) en présence de bis(pentane-2,4-dionoto)calcium) / triéthylamine.**

Laprolat E-181 est une résine dicyclocarbonate préparée à partir d'un di-époxy oligoéthers et commercialée par MACROMER® Ltd. Elle a un poids moléculaire de 400 g/mol, le pourcentage massique en cylocarbonate représentant 44,5% en poids.

Laprolat ETF est une résine de type tricylocarbonate-(oligoéther-oxyphenyl)méthane qui est commercialisée par la société MACROMER® Ltd. Elle a un poids moléculaire de MW =750 g/mol, le pourcentage massique en cylocarbonate représentant 44,5% en poids.

Dans un bécher, on a introduit le Laprolat E-181 (16,3 g, 40,75 mmol) et le Laprolat ETF (4,0 g, 5,33 mmol). L'ensemble est porté à 90°C pendant 4h pour obtenir un mélange liquide homogène. Au mélange revenu à température ambiante sont ajoutés sous agitation le bis(pentane-2,4-dionato)calcium (200 mg, 0,84 mmol , Siccanor), la triéthylamine (84,9 mg, 0,84 mmol, Sigma Aldrich®), la triméthylhexaméthylène diamine (7,7 g, 48,6 mmol), Vestamin® TMD Evonik Degussa GmbH). Ensuite, l'évolution de la rigidité du milieu réactionnel au cours du temps a été réalisée à température ambiante, au moyen d'un dispositif TROMBOMAT® (commercialisé par la société PRODEMAT® S.A.) qui est capable de déterminer le passage au point de gel.

Il a été constaté un temps de gel très court inférieur à 1 minute.

Exemple 9 : Préparation d'un hybride époxy / polyuréthane hydroxylé

Dans un bécher on a introduit à pression atmosphérique, à température ambiante, et sous agitation mécanique, le carbonate de propylène (0,8g , 7,8 mmol, Huntsman®), le carbonate de glycerol (3,0g, 25,4 mmol, Huntsman®), le Laprolat® ETF (2,0g, 2,7mmol, MACROMER®) , le triépoxy YH® 300 (12,5g, EEW= 142,5 g/eq , Kukdo®), le bis-(acétylacétonate) de calcium(II) (200 mg , 0,84 mmol, Siccanor®), la triéthyle amine (54,5 mg , 0,55 mmol Sigma Aldrich®), la Dolomie® DRB 3 (3,6g, oxyde double calcium magnésium, Imerys® Performance minerals). Le mélange chargé est laissé sous agitation 1 h à température ambiante.

Puis on a ajouté la triméthylhexaméthylène diamine « vestamine® TMD » (3,26g, 20,63 mmol, Evonik Degussa GmbH). Le mélange est laissé à température ambiante 1 h, puis porté 30 minutes à 100°C pour finir la polymérisation/réticulation et conférer au matériaux ses propriétés mécaniques finales.

La Dolomie DRB 3 a été utilisée en tant que charge minérale.

Le polyhydroxyuréthane hybride ainsi obtenu présente les propriétés suivantes:

- Dureté à 23 °C (Shore A 70, selon la norme Iso 868);
- Force maximale à la rupture 160N (Norme Iso R 527);
- Déformation maximale à la rupture 38,22% (Norme Iso R 527)
- Module d'Young : 8,27 MPa,
- Reprise hydrique réalisée par immersion 1 h dans eau bouillante 2,2%.

## Revendications

1. Procédé de préparation d'un composé comprenant au moins un motif β-hydroxy-uréthane et/ou au moins un motif γ-hydroxy-uréthane, consistant à faire réagir un composé A comprenant au moins un motif réactif cyclocarbonate avec un composé B comprenant au moins un motif réactif amino ($-NH_2$) en présence d'un catalyseur, ledit procédé étant **caractérisé en ce que** ledit catalyseur comprend au moins un complexe organométallique et un co-catalyseur choisi dans le groupe des bases de Lewis et/ou des sels de tétra-alkyl ammonium.

2. Procédé selon la revendication 1, **caractérisé en ce que** le complexe organométallique contient un métal alcalin, alcalino-terreux ou de transition choisi parmi les groupes IA, IIA, IIIA, IIIB, IVA, IVB, VB, VIB, VIIB et VIII du tableau périodique, et au moins deux ligands β-dicétonate.

3. Procédé selon la revendication 2, **caractérisé en ce que** les ligands β-dicétonate sont choisis dans la liste suivante: acétylacétonato, 1, 1, 1, 5, 5, 5-hexa-fluoro-acétylacétonate, 1,1,1-trifluoro-acétylacétonate, 1,1,1-trifluoro-5,5-diméthylacétylacétonate et 2, 2, 6, 6-tetraméthyl-3, 5-heptanedionate.

4. Procédé selon la revendication 1 ou 2, **caractérisé en ce que** le complexe organométallique est choisi dans la liste suivante : tris(acétylacétonate) d'aluminum(III), tris(hexafluoroacétylacétonate) d'aluminium(III), tris(trifluoroacétylacétonate) d'aluminium(III), tris(2,2,6,6-tétraméthyl-3,5-heptanedionato)aluminium(III), bis(acétylacétonate) de calcium(II), tris(acétylacétonate) de chrome(III), tris(2, 2, 6, 6-tétraméthyl-3, 5-heptanedionate) de chrome(III), tris(acétylacétonate) de cobalt(III), tris(nitro-acétylacétonate) de cobalt(III), tris(2,2,6,6-tétraméthyl-3,5-heptanedionate) de cobalt(III), bis(acétylacétonate) de cuivre(II), bis(2,2,6,6- tétraméthyl -3, 5-heptanedionate) de cuivre(II), tris(acétylacétonato)gallium (III), acétylacétonate d'hafnium(IV), tris(acétylacétonato)indium(III), tris(acétylacétonate) de fer(III), tris(2,2,6,6- tétraméthyl -3,5-heptanedionate) de fer(III), tris(acétylacétonate) de manganèse (III), bis(acétylacétonate) de nickel(II), bis(acétylacétonate) de palladium(II), bis(trifluoroacétylacétonate) de palladium(II), acétylacétonate de sodium, bis(acétylacétonate) d'oxyde de titane (IV), bis(2, 2, 6, 6-tétraméthyl-3, 5-heptanedionate) d'oxyde de titane(IV), tris(2,2,6,6-tétraméthyl-3,5-heptanedionato) de titane(III), tris(2, 2, 6, 6-tétraméthyl-3, 5-heptanedionato) oxotitane(IV), dichloro-bis-(2,2,6,6-tétraméthyl-3,5-,heptanedionato)titanium(IV), le (diisopropoxyde)bis(acétylacétonate) de titane(IV), di(isopropoxide)bis(2,2,6,6-tétraméthyl-3,5-heptanedionato)titanium(IV), bis(acétylacétonate) de zinc(II), tetrakis(acétylacétonate)zirconium(IV), tetrakis(hexafluoroacétylacétonate) de zirconium(IV), tetrakis(2,2,6,6-tétraméthyl-3,5-heptanedionato)zirconium(IV), tetrakis(trifluoroacétylacétonate) de zirconium(IV).

5. Procédé selon les revendications précédentes, **caractérisé en ce que** le rapport molaire complexe organométallique/composé A est compris entre 0,001 à 0,05, de préférence entre 0.002 et 0,01.

6. Procédé selon les revendications précédentes, **caractérisé en ce que** le co-catalyseur est choisi parmi les trialkylamines, les amines hétérocycliques aromatiques, les trialkylphosphines, les triarylphosphines, les trialkylphosphites, les triarylphosphites, les sels de tetralakyl ammoniums, ou leurs mélanges.

7. Procédé selon les revendications précédentes, **caractérisé en ce que** le co-catalyseur est utilisé à raison de 1,5 à 3 moles par mole de complexe organométallique, de préférence à 2 moles par mole de complexe organométallique.

8. Procédé selon la revendication 1, **caractérisé en ce que** le composé A est choisi dans le groupe des composés, oligomères ou polymères terminés par au moins un cyclocarbonate, de préférence au moins deux cyclocarbonates.

9. Procédé selon les revendications précédentes, **caractérisé en ce que** le composé A est un composé répondant à :

- la formule générale A1 :

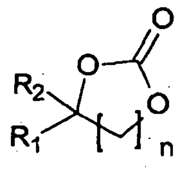

**A1**

dans laquelle :
- n représente un nombre entier égal à 1 ou 2,
- $R_1$ représente un atome d'hydrogène ou un groupe alkyle en $C_{1-6}$, linéaire ou ramifié, saturé ou insaturé, et le cas échéant, substitué par un ou plusieurs substituants choisis parmi halogène, hydroxy, cyano, carboxy, trifluorométhyl, alkyl en $C_{1-6}$, alkylamino en $C_{1-6}$, dialkylamino en $C_{1-6}$, alcoxy en $C_{1-6}$, alkylcarboxylate en $C_{1-6}$,
- $R_2$ représente un atome d'hydrogène ou un groupe choisi parmi : un alkyle en $C_{1-20}$, linéaire ou ramifié, saturé ou insaturé, et dans lequel des unités méthylènes non voisines peuvent être remplacées par des radicaux -O-, -S-,-S(O)-, $-SO_2-$, -O-C(=O)-, $-N(R^{21})-$, $-N(R^{21})-C(=O)-$, $-N(R^{21})-C(=O)-O-$ $N(R^{21})-C(=O)-N(R^{22})-$, avec $R^{21}$ et $R^{22}$ identiques ou différents, représentant des atomes d'hydrogène ou des radicaux choisis parmi alkyl en $C_{1-6}$ ou aryle en $C_{6-14}$; un cycloalkyle en $C_{3-10}$: un aryle en $C_{6-14}$; un hétérocyle en $C_{3-10}$ portant un ou plusieurs hétéroatomes choisis parmi N, O, S; un hétéroaryl en $C_{4-13}$ portant un ou plusieurs hétéroatomes choisis parmi N, O, S ; les membres de ce groupe étant éventuellement substitués par un ou plusieurs substituants choisis parmi halogène, hydroxy, cyano, carboxy, trifluorométhyl, alkyl en $C_{1-6}$, alkylamino en $C_{1-6}$, dialkylamino en $C_{1-6}$, alcoxy en $C_{1-6}$, alkylcarboxylate en $C_{1-6}$,
- ou, la formule générale A2 :

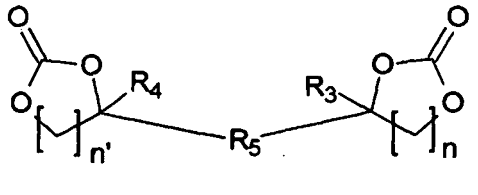

**A2**

dans laquelle :

- n, n', identiques ou différents représentent un nombre entier égal à 1 ou 2,
- **$R_3$** et **$R_4$,** identiques ou différents, représentent un atome d'hydrogène ou un groupe alkyle en $C_{1-8}$, linéaire ou ramifié, saturé ou insaturé, et le cas échéant, substitués par un ou plusieurs substituants choisis parmi halogène, hydroxy, cyano, carboxy, trifluorométhyl, alkyl en $C_{1-6}$, alkylamino en $C_{1-6}$, dialkylamino en $C_{1-6}$, alcoxy en $C_{1-6}$, alkylcarboxylate en $C_{1-6}$,
- **$R_5$** représente:

une liaison directe,

■ un groupe bivalent choisi parmi : un alkylène en $C_{1-20}$, linéaire ou ramifié, saturé ou insaturé, et dans lequel des unités méthylènes non voisines peuvent être remplacées par des radicaux -O-, -S-, -S(O)- -$SO_2$-, -C(=O)-O-, -N($R^{21}$)-,-N($R^{21}$)-C(=O)-, -N($R^{21}$)-C(=O)-O-, -N($R^{21}$)-C(=O)-N($R^{22}$)-, avec $R^{21}$ et $R^{22}$ identiques ou différents, représentant des atomes d'hydrogène ou des radicaux choisis parmi alkyl en $C_{1-6}$ ou aryle en $C_{6-14}$; un cycloalkylène en $C_{3-10}$; un arylène en $C_{6-14}$; un hétérocyclène en $C_{3-10}$ portant un ou plusieurs hétéroatomes choisis parmi N, O, S; un hétéroarylène $C_{4-13}$ portant un ou plusieurs hétéroatomes choisis parmi N, O, S; les membres de ce groupe étant éventuellement substitués par un ou plusieurs substituants choisis parmi halogène, hydroxy, cyano, carboxy, trifluorométhyl, alkyl en $C_{1-6}$, alkylamino en $C_{1-6}$, dialkylamino en $C_{1-6}$, alcoxy en $C_{1-6}$, alkylcarboxylate en $C_{1-6}$,

■ ou, un groupe bivalent présentant la formule générale I suivante :

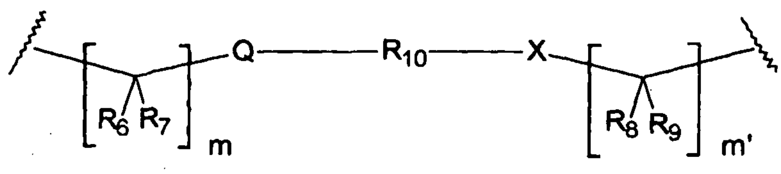

**(I)**

dans laquelle :

■ m et m' identiques ou différents désignent un nombre entier compris entre 1 à 100,

■ $R_6$, $R_7$, $R_8$ et $R_9$, identiques ou différents, représentent des atomes d'hydrogène ou des radicaux alkyles en $C_{1-6}$, linéaires ou ramifiés, saturés ou insaturés, et le cas échéant, substitués par un ou plusieurs substituants choisis parmi halogène, hydroxy, cyano, carboxy, trifluorométhyl, alkyl en $C_{1-6}$, alkylamino en $C_{1-6}$, dialkylamino en $C_{1-6}$, alcoxy en $C_{1-6}$, alkylcarboxylate en $C_{1-6}$,

■ Q et X identiques ou différents représentent un groupe -O-C(=O)-,-N($R^{21}$)-, -N($R^{21}$)-C(=O)-, -N($R^{21}$)-C(=O)-O-, -N($R^{21}$)-C(=O)-N($R^{22}$)-, avec $R^{21}$ et $R^{22}$ sont tels que définis précédemment,

■ $R_{10}$ représente un groupe bivalent choisi parmi : un alkylène en $C_{1-20}$, linéaire ou ramifié, saturé ou non, et dans lequel des unités méthylènes non voisines peuvent être remplacées par des radicaux -O-, -S-, -S(O)- -$SO_2$-,-C(=O)-O-, -N($R^{21}$)-, -N($R^{21}$)-C(=O)-, -N($R^{21}$)-C(=O)-O-, -N($R^{21}$)-C(=O)-N($R^{22}$)-, avec $R^{21}$ et $R^{22}$ identiques ou différents, représentant des atomes d'hydrogène ou des radicaux choisis parmi alkyl en $C_{1-6}$ ou aryle en $C_{6-14}$; un cycloalkylène en $C_{3-10}$; un arylène en $C_{6-14}$; un hétérocyclène en $C_{3-10}$ portant un ou plusieurs hétéroatomes choisis parmi N, O, S; un hétéroarylène $C_{4-13}$ portant un ou plusieurs hétéroatomes choisis parmi N, O, S; les membres de ce groupe bivalent étant éventuellement substitués par un ou plusieurs substituants choisis parmi halogène, hydroxy, cyano, carboxy, trifluorométhyl, alkyl en $C_{1-6}$, alkylamino en $C_{1-6}$, dialkylamino en $C_{1-6}$, alcoxy en $C_{1-6}$, alkylcarboxylate en $C_{1-6}$;

■ ou, un groupe répondant à l'une des formules II ou III :

$$-(CH_2\text{-}CH_2\text{-}O)_t\text{-}CH_2\text{-}\ CH_2\text{-} \qquad (II),$$

ou

$$-C(=O)-CH_2-O-(CH_2-CH_2-O)_t-CH_2-C(=O)- \quad (III)$$

dans lesquelles :
- t, t', identiques ou différents, désignent un nombre entier compris entre 1 et 100.

**10.** Procédé selon les revendications précédentes, **caractérisé en ce que** le composé **A** est un composé **A3** obtenu par :

- la réaction du 4-hydroxyméthyl-1,3-dioxolan-2-one avec un composé comprenant deux ou plusieurs fonctions isocyantes ,
- la réaction d'estérification du 4-hydroxyméthyl-1,3-dioxolan-2-one avec un composé comprenant une ou plusieurs fonctions acides carboxyliques,
- la réaction du triglycidyl isocyanurate avec le dioxyde de carbone, ou
- la réaction d'un composé comprenant deux ou plusieurs groupes éthers de glycidyle avec le dioxyde de carbone.

**11.** Procédé selon la revendication 1, **caractérisé en ce que** le composé **B** est choisi dans le groupe des composés, oligomères ou polymères terminés par au moins un motif réactif ($-NH_2$), de préférence par au moins deux motifs réactifs ($-NH_2$).

**12.** Procédé selon la revendication 1 ou la revendication 11, **caractérisé en ce que** le ou les motifs réactifs (-NH2) sont portés par des carbones saturés (sp3), lesquels sont également porteurs d'au moins un hydrogène, de préférence de deux hydrogènes.

**13.** Procédé selon les revendications précédentes, **caractérisé en ce que** le composé **B** comprenant au moins un motif réactif amino ($-NH_2$) est choisi dans la liste suivante : butylamine ; hexylamine ; cyclohexylamine ; éthanolamine ; propanolamine ; éthylène diamine ; propylène diamine ; butylène diamine ; pentaméthylène diamine ; hexaméthylène diamine ; heptaméthylène diamine ; tétraméthylène diamine ; octaméthylène diamine ; nonaméthylène diamine ; décaméthylène diamine ; méthyl-2-pentaméthylène diamine ; undécaméthylène diamine ; dodécaméthylène diamine ; xylylène diamine ; isophorone diamine ; triméthyl hexaméthylène diamine ; 1,2-diaminocyclohexane ; 1,4-diaminocyclohexane ; 4,4'-diaminocyclohexyl méthane ; 2-(2-aminoéthoxy)éthanol ; bis-(3-méthyl4-aminocyclohexyl)méthane ; 2,2-bis-(4-aminocyclohexyl)propane ; nitrile tri(éthaneamine) ; bis-(3-aminopropyl)méthylamine ; 3-amino-1-(méthylamino)propane ; 3-amino-1-(cyclohexylamino)propane ; N-(2-hydroxyéthyl)éthylène diamine ; 4,7-dioxadécane-1,10-diamine ; 4,9-dioxadodécane-1,12-diamine; 7-méthyl 4,10-dioxatridécane-1 ,13-diamine; une polyéther monoamine de préférence choisie parmi l'hydroxy polyéthyléne glycol amine et la méthoxypolyéthyléne glycol amine, une polyéther diamine, de préférence choisie parmi une polyoxyéthylène diamine et une polyoxypropylène diamine ; et/ou un oligomère ou polymère terminé par au moins un motif réactif amino ($-NH_2$), de préférence par deux ou plusieurs motifs réactifs amino ($-NH_2$), ledit oligomère ou polymère pouvant avoir un squelette polyamide, polyéther et/ou polyester.

**14.** Procédé selon les revendications précédentes, **caractérisé en ce que** le composé A et le composé **B** sont mélangés de manière à ce que le rapport molaire composé **B** sur composé **A** soit compris entre 0,5 et 2, de préférence égal à 1.

**15.** Procédé selon les revendications précédentes, **caractérisé en ce que** le composé **A** et le composé **B** sont préalablement mélangés avec un plastifiant avant la mise en contact avec le catalyseur, lequel catalyseur comprend au moins le complexe organométallique et le co-catalyseur.

**16.** Utilisation d'un catalyseur comprenant au moins un complexe organométallique et un co-catalyseur choisi dans le groupe des bases de Lewis et/ou des sels de tétra-alkyl ammonium, pour catalyser la réaction de condensation entre un composé **A** comprenant au moins un motif réactif cyclocarbonate et un composé **B** comprenant au moins un motif réactif amino ($-NH_2$).

## Patentansprüche

**1.** Verfahren zur Herstellung einer Verbindung mit mindestens einer β-Hydroxyurethan-Einheit und/oder mindestens

einer γ-Hydroxyurethan-Einheit, bei dem man eine Verbindung A mit mindestens einer reaktiven Cyclocarbonat-Einheit in Gegenwart eines Katalysators mit einer Verbindung B mit mindestens einer reaktiven Amino-Einheit ($-NH_2$) umsetzt, wobei das Verfahren **dadurch gekennzeichnet ist, dass** der Katalysator mindestens einen metallorganischen Komplex und einen Cokatalysator aus der Gruppe der Lewis-Basen und/oder Tetraalkylammoniumsalze umfasst.

2. Verfahren nach Anspruch 1, **dadurch gekennzeichnet, dass** der metallorganische Komplex ein Alkali-, Erdalkali- oder Übergangsmetall aus den Gruppen IA, IIA, IIIA, IIIB, IVA, IVB, VB, VIB, VIIB und VIII des Periodensystems und mindestens zwei β-Diketonat-Liganden enthält.

3. Verfahren nach Anspruch 2, **dadurch gekennzeichnet, dass** die β-Diketonat-Liganden aus der folgenden Liste ausgewählt werden: Acetylacetonato, 1,1,1,5,5,5-Hexafluoracetylacetonat, 1,1,1-Trifluoracetylacetonat, 1,1,1-Trifluor-5,5-dimethylacetylacetonat und 2,2,6,6-Tetramethyl-3,5-heptandionat.

4. Verfahren nach Anspruch 1 oder 2, **dadurch gekennzeichnet, dass** der metallorganische Komplex aus der folgenden Liste ausgewählt wird:

   Aluminium(III)-tris(acetylacetonat), Aluminium(III)-tris(hexafluoracetylacetonat), Aluminium(III)-tris(trifluoracetylacetonat), Tris (2,2,6,6-tetramethyl-3,5-heptandionato)aluminium(III), Calcium(II)-bis(acetylacetonat), Chrom(III)-tris(acetyl-acetonat), Chrom(III)-tris(2,2,6,6-tetramethyl-3,5-heptandionat), Cobalt (III)-tris(acetylacetonat), Cobalt(III)-tris(nitroacetylacetonat), Cobalt (III) -tris (2,2,6,6-tetramethyl-3,5-heptandionat), Kupfer(II)-bis(acetylacetonat), Kupfer(II)-bis(2,2,6,6-tetramethyl-3,5-heptandionat), Tris(acetylacetonato)gallium(III), Hafnium(IV)-acetylacetonat, Tris (acetylacetonato)-indium(III), Eisen (III)-tris (acetylacetonat), Eisen(III)-tris(2,2,6,6-tetramethyl-3,5-heptandionat), Mangan (III) -tris (acetylacetonat), Nickel (II) -bis (acetylacetonat), Palladium (II)-bis (acetylacetonat), Palladium (II) -bis (trifluor-acetylacetonat), Natriumacetylacetonat, Titan(IV)-oxid-bis(acetylacetonat), Titan(IV)-oxid-bis-(2,2,6,6-tetramethyl-3,5-heptandionat), Titan-(III) -tris(2,2,6,6-tetramethyl-3,5-heptandionat), Tris(2,2,6,6-tetramethyl-3,5-heptandionat)oxo-titan(IV), Dichlorobis(2,2,6,6-tetramethyl-3,5-heptandionat)titan(IV), Titan(IV)-(diisopropoxid)-bis(acetylacetonat), Di(isopropoxid) bis(2,2,6,6-tetramethyl-3,5-heptandionato)titan(IV), Zink(II)-bis (acetylacetonat), Zirconium(IV)-tetrakis-(acetylacetonat), Zirconium(IV)-tetrakis-(hexafluoracetylacetonat), Tetrakis(2,2,6,6-tetramethyl-3,5-heptandionato)zirconium(IV), Zirconium(IV)-tetrakis(trifluoracetylacetonat).

5. Verfahren nach den vorhergehenden Ansprüchen, **dadurch gekennzeichnet, dass** das Molverhältnis von metallorganischem Komplex zu Verbindung A zwischen 0,001 und 0,05 und vorzugsweise zwischen 0,002 und 0,01 liegt.

6. Verfahren nach den vorhergehenden Ansprüchen, **dadurch gekennzeichnet, dass** der Cokatalysator aus Trialkylaminen, aromatischen heterocyclischen Aminen, Trialkylphosphinen, Triarylphosphinen, Trialkylphosphiten, Triarylphosphiten, Tetraalkyl-ammoniumsalzen oder Mischungen davon ausgewählt wird.

7. Verfahren nach den vorhergehenden Ansprüchen, **dadurch gekennzeichnet, dass** der Cokatalysator in einer Menge von 1,5 bis 3 mol pro Mol metallorganischer Komplex, vorzugsweise von 2 mol pro Mol metallorganischer Komplex, verwendet wird.

8. Verfahren nach Anspruch 1, **dadurch gekennzeichnet, dass** die Verbindung A aus der Gruppe der Verbindungen, Oligomere oder Polymere, die durch mindestens ein Cyclocarbonat, vorzugsweise mindestens zwei Cyclocarbonate, terminiert sind, ausgewählt wird.

9. Verfahren nach den vorhergehenden Ansprüchen, **dadurch gekennzeichnet, dass** es sich bei der Verbindung A um eine Verbindung handelt, die: - der allgemeinen Formel Al :

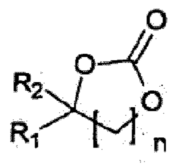

**A1**

in der:

- n für eine ganze Zahl mit einem Wert von 1 oder 2 steht,
- $R_1$ für ein Wasserstoffatom oder eine gesättigte oder ungesättigte, lineare oder verzweigte $C_{1-6}$-Alkylgruppe, die gegebenenfalls durch einen oder mehrere Substituenten, die aus Halogen, Hydroxy, Cyano, Carboxy, Trifluormethyl, $C_{1-6}$-Alkyl, $C_{1-6}$-Alkylamino, Di-$C_{1-6}$-alkylamino, $C_{1-6}$-Alkoxy und $C_{1-6}$-Alkylcarboxylat ausgewählt sind, substituiert ist, steht,
- $R_2$ für ein Wasserstoffatom oder eine Gruppe, die aus einem gesättigten oder ungesättigten, linearen oder verzweigten $C_{1-20}$-Alkyl, in dem nicht benachbarte Methylen-Einheiten durch -O-, -S-, -S(O)-, -$SO_2$-, -O-C(=O)-, -N($R^{21}$) -, -N($R^{21}$) -C(=O)-, -N($R^{21}$)-C(=O)-O-, -N($R^{21}$)-C(=O)-N($R^{22}$)-Reste ersetzt sein können, wobei $R^{21}$ und $R^{22}$ gleich oder verschieden sind und für Wasserstoffatome oder aus $C_{1-6}$-Alkyl oder $C_{6-14}$-Aryl ausgewählte Reste stehen; einem $C_{3-10}$-Cycloalkyl; einem $C_{6-14}$-Aryl; einem $C_{3-10}$-Heterocyclus mit einem oder mehreren aus N, 0 und S ausgewählten Heteroatomen und einem $C_{4-13}$-Heteroaryl mit einem oder mehreren aus N, 0 und S ausgewählten Heteroatomen ausgewählt ist, steht; wobei die Mitglieder dieser Gruppe gegebenenfalls durch einen oder mehrere Substituenten, die aus Halogen, Hydroxy, Cyano, Carboxy, Trifluormethyl, $C_{1-6}$-Alkyl, $C_{1-6}$-Alkylamino, Di-$C_{1-6}$-alkylamino, $C_{1-6}$-Alkoxy und $C_{1-6}$,-Alkylcarboxylat ausgewählt sind, substituiert sind,
- oder der allgemeinen Formel A2:

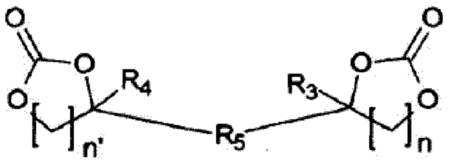

A2

in der:
- n und n' gleich oder verschieden sind und für eine ganze Zahl mit einem Wert von 1 oder 2 stehen,
- $R_3$ und $R_4$ gleich oder verschieden sind und für ein Wasserstoffatom oder eine gesättigte oder ungesättigte, lineare oder verzweigte $C_{1-6}$-Alkylgruppe, die gegebenenfalls durch einen oder mehrere Substituenten, die aus Halogen, Hydroxy, Cyano, Carboxy, Trifluormethyl, $C_{1-6}$-Alkyl, $C_{1-6}$-Alkylamino, Di-$C_{1-6}$-alkylamino, $C_{1-6}$-Alkoxy und $C_{1-6}$-Alkylcarboxylat ausgewählt sind, substituiert ist, stehen,
- $R_5$ für:

  ■ eine direkte Bindung,
  ■ eine zweiwertige Gruppe, ausgewählt aus: einem gesättigten oder ungesättigten, linearen oder verzweigten $C_{1-20}$-Alkylen, in dem nicht benachbarte Methylen-Einheiten durch -O-, -S-, -S(0)-, -$SO_2$-, -C(=O)-O-, -N($R^{21}$) -, -N($R^{21}$)-C(=O)-, -N($R^{21}$)-C(=O)-O-, -N($R^{21}$) -C(=O)-N($R^{22}$) -Reste ersetzt sein können, wobei $R^{21}$ und $R^{22}$ gleich oder verschieden sind und für Wasserstoffatome oder aus $C_{1-6}$-Alkyl oder $C_{6-14}$-Aryl ausgewählte Reste stehen; einem $C_{3-10}$-Cycloalkylen; einem $C_{6-14}$-Arylen; einem $C_{3-10}$-Heterocyclen mit einem oder mehreren aus N, 0 und S ausgewählten Heteroatomen und einem $C_{4-13}$-Heteroarylen mit einem oder mehreren aus N, 0 und S ausgewählten Heteroatomen; wobei die Mitglieder dieser Gruppe gegebenenfalls durch einen oder mehrere Substituenten, die aus Halogen, Hydroxy, Cyano, Carboxy, Trifluormethyl, $C_{1-6}$-Alkyl, $C_{1-6}$-Alkylamino, Di-$C_{1-6}$-alkylamino, $C_{1-6}$-Alkoxy und $C_{1-6}$-Alkylcarboxylat ausgewählt sind, substituiert sind,
  ■ oder eine zweiwertige Gruppe der folgenden allgemeinen Formel I:

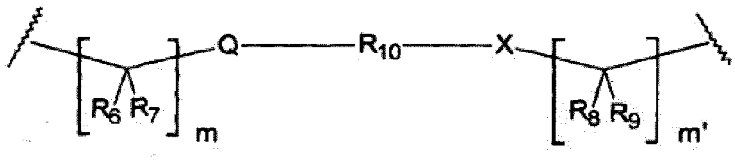

(I)

in der:

■ m und m' gleich oder verschieden sind und für eine ganze Zahl zwischen 1 und 100 stehen,
■ $R_6$, $R_7$, $R_8$ und $R_9$ gleich oder verschieden sind und für Wasserstoffatome oder gesättigte oder ungesättigte, lineare oder verzweigte $C_{1-6}$-Alkylreste, die gegebenenfalls durch einen oder mehrere Substituenten, die aus Halogen, Hydroxy, Cyano, Carboxy, Trifluormethyl, $C_{1-6}$-Alkyl, $C_{1-6}$-Alkylamino, Di-$C_{1-6}$-alkylamino, $C_{1-6}$-Alkoxy und $C_{1-6}$-Alkylcarboxylat ausgewählt sind, substituiert sind, stehen,
■ Q und X gleich oder verschieden sind und für eine -O-C(=O)-, -N($R^{21}$)-, -N($R^{21}$)-C(=O)-, -N($R^{21}$)-C(=O)-O- oder -N($R^{21}$))-C(=O)-N($R^{22}$)-Gruppe stehen, wobei $R^{21}$ und $R^{22}$ wie oben definiert sind,
■ $R_{10}$ für eine zweiwertige Gruppe, ausgewählt aus: einem gesättigten oder ungesättigten, linearen oder verzweigten $C_{1-20}$-Alkylen, in dem nicht benachbarte Methylen-Einheiten durch -O-, -S-, -S(O)-, -$SO_2$-, -C(=O)-O-, -N($R^{21}$) -, -N($R^{21}$) -C(=O)-, -N($R^{21}$)-C (=O)-O-, -N($R^{21}$)-C(=O)-N($R^{22}$)-Reste ersetzt sein können, wobei $R^{21}$ und $R^{22}$ gleich oder verschieden sind und für Wasserstoffatome oder aus $C_{1-6}$-Alkyl oder $C_{6-14}$-Aryl ausgewählte Reste stehen; einem $C_{3-10}$-Cycloalkylen; einem $C_{6-14}$-Arylen; einem $C_{3-10}$-Heterocyclen mit einem oder mehreren aus N, 0 und S ausgewählten Heteroatomen und einem $C_{4-13}$-Heteroarylen mit einem oder mehreren aus N, 0 und S ausgewählten Heteroatomen; steht; wobei die Mitglieder dieser Gruppe gegebenenfalls durch einen oder mehrere Substituenten, die aus Halogen, Hydroxy, Cyano, Carboxy, Trifluormethyl, $C_{1-6}$-Alkyl, $C_{1-6}$-Alkylamino, Di-$C_{1-6}$-alkylamino, $C_{1-6}$-Alkoxy und $C_{1-6}$,-Alkylcarboxylat ausgewählt sind, substituiert sind,
■ oder eine Gruppe, die einer der Formeln II oder III entspricht:

$$-(CH_2-CH_2-O)_t-CH_2-CH_2- \qquad (II)$$

oder

$$-C(=)-CH_2-O-(CH_2-CH_2-O)_{t'}-CH_2-C(=O)- \qquad (III)$$

in denen:

- t und t' gleich oder verschieden sind und für eine ganze Zahl zwischen 1 und 100 stehen; steht; entspricht.

**10.** Verfahren nach den vorhergehenden Ansprüchen, **dadurch gekennzeichnet, dass** es sich bei der Verbindung A um eine durch:

- Umsetzung von 4-Hydroxymethyl-1,3-dioxolan-2-on mit einer Verbindung mit zwei oder mehr Isocyanatfunktionen,
- Veresterungsreaktion von 4-Hydroxymethyl-1,3-dioxolan-2-on mit einer Verbindung mit einer oder mehreren Carbonsäurefunktionen,
- Umsetzung von Triglycidylisocyanurat mit Kohlendioxid oder
- Umsetzung einer Verbindung mit zwei oder mehr Glycidylethergruppen mit Kohlendioxid erhaltene Verbindung A3 handelt.

**11.** Verfahren nach Anspruch 1, **dadurch gekennzeichnet, dass** die Verbindung B aus der Gruppe der Verbindungen, Oligomere oder Polymere, die durch mindestens eine reaktive Einheit (-$NH_2$), vorzugsweise durch mindestens zwei reaktive Einheiten (-$NH_2$), terminiert sind, ausgewählt wird.

**12.** Verfahren nach Anspruch 1 oder Anspruch 11, **dadurch gekennzeichnet, dass** die reaktive(n) Einheit(en) (-$NH_2$) an gesättigten Kohlenstoffatomen (sp3) stehen, die auch mindestens ein Wasserstoffatom, vorzugsweise zwei Wasserstoffatome, tragen.

**13.** Verfahren nach einem der vorhergehenden Ansprüche, **dadurch gekennzeichnet, dass** die Verbindung B mit mindestens einer reaktiven Amino-Einheit (-$NH_2$) aus der folgenden Liste ausgewählt ist: Butylamin; Hexylamin; Cyclohexylamin; Ethanolamin; Propanolamin; Ethylendiamin; Propylendiamin; Butylendiamin; Pentamethylendiamin; Hexamethylendiamin; Heptamethylendiamin; Tetramethylendiamin; Octamethylendiamin; Nonamethylendiamin; Decamethylendiamin; 2-Methylpentamethylendiamin; Undecamethylendiamin; Dodecamethylendiamin; Xylylendiamin; Isophorondiamin; Trimethylhexamethylendiamin; 1,2-Diaminocyclohexan; 1,4-Diaminocyclohexan; 4,4'-Diaminocyclohexylmethan; 2-(2-Aminoethoxy)ethanol; Bis(3-methyl-4-aminocyclohexyl)methan; 2,2-Bis(4-aminocyclohexyl)propan; Nitriltri(ethanamin); Bis(3-aminopropyl)methylamin; 3-Amino-1-(methylamino)propan; 3-Amino-1-(cyclohexylamino)propan; N-(2-Hydroxyethyl)-ethylendiamin; 4,7-Dioxadecan-1,10-diamin; 4,9-Dioxadodecan-1,12-diamin; 7-Methyl-4,10-dioxatridecan-1,13-diamin; einem Polyethermonoamin, vorzugsweise ausgewählt aus Hydroxypolyethylenglykolamin und Methoxypolyethylenglykolamin, einem Polyetherdiamin, vorzugsweise ausgewählt aus einem Polyoxyethylendiamin und einem Polyoxypropylendiamin; und/oder einem Oligomer oder Polymer, das durch mindestens eine reaktive Amino-Einheit ($-NH_2$), vorzugsweise durch zwei oder mehr reaktive Amino-Einheiten ($-NH_2$), terminiert ist, wobei das Oligomer oder Polymer ein Polyamid-, Polyether- und/oder Polyestergerüst aufweisen kann.

**14.** Verfahren nach den vorhergehenden Ansprüchen, **dadurch gekennzeichnet, dass** die Verbindung A und die Verbindung B so gemischt werden, dass das Molverhältnis von Verbindung B zu Verbindung A zwischen 0,5 und 2 liegt und vorzugsweise gleich 1 ist.

**15.** Verfahren nach den vorhergehenden Ansprüchen, **dadurch gekennzeichnet, dass** die Verbindung A und die Verbindung B vor dem Inberührungbringen mit dem Katalysator mit einem Weichmacher vorgemischt werden, wobei der Katalysator mindestens den metallorganischen Komplex und den Cokatalysator umfasst.

**16.** Verwendung eines Katalysators, der mindestens einen metallorganischen Komplex und einen Cokatalysator aus der Gruppe der Lewis-Basen und/oder Tetraalkylammoniumsalze umfasst, für die Katalyse der Kondensationsreaktion zwischen einer Verbindung A mit mindestens einer reaktiven Cyclocarbonat-Einheit und einer Verbindung B mit mindestens einer reaktiven Amino-Einheit ($-NH_2$).

## Claims

**1.** Process for preparing a compound comprising at least one β-hydroxyurethane unit and/or at least one γ-hydroxyurethane unit, which consists in reacting a compound A comprising at least one cyclocarbonate reactive unit with a compound B comprising at least one amino ($-NH_2$) reactive unit in the presence of a catalyst, said process being **characterized in that** said catalyst comprises at least one organometallic complex and a cocatalyst chosen from the group of Lewis bases and/or tetraalkylammonium salts.

**2.** Process according to Claim 1, **characterized in that** the organometallic complex contains an alkali metal, alkaline-earth metal or transition metal chosen from groups IA, IIA, IIIA, IIIB, IVA, IVB, VB, VIB, VIIB and VIII of the Periodic Table, and at least two β-diketonate ligands.

**3.** Process according to Claim 2, **characterized in that** the β-diketonate ligands are chosen from the following list: acetylacetonato, 1,1,1,5,5,5-hexafluoroacetylacetonate, 1,1,1-trifluoroacetylacetonate, 1,1,1-trifluoro-5,5-dimethylacetylacetonate and 2,2,6,6-tetramethyl-3,5-heptanedionate.

**4.** Process according to Claim 1 or 2, **characterized in that** the organometallic complex is chosen from the following list: aluminium(III) tris(acetylacetonate), aluminium(III) tris (hexafluoroacetylacetonate), aluminium(III) tris (trifluoroacetylacetonate), tris (2,2,6,6-tetramethyl-3,5-heptanedionato)aluminium(III), calcium(II) bis(acetylacetonate), chromium(III) tris(acetylacetonate), chromium(III) tris(2,2,6,6-tetramethyl-3,5-heptanedionate), cobalt(III) tris(acetylacetonate), cobalt(III) tris (nitroacetylacetonate), cobalt(III) tris(2,2,6,6-tetramethyl-3,5-heptanedionate), copper(II) bis (acetylacetonate) , copper(II) bis(2,2,6,6-tetramethyl-3,5-heptanedionate), tris(acetylacetonato)gallium(III), hafnium(IV) acetylacetonate, tris (acetylacetonato)indium(III), iron(III) tris(acetylacetonate), iron(III) tris (2,2,6,6-tetramethyl-3,5-heptanedionate), manganese (III) tris(acetylacetonate), nickel(II) bis(acetylacetonate), palladium(II) bis(acetylacetonate), palladium(II) bis (trifluoroacetylacetonate), sodium acetylacetonate, titanium(IV) oxide bis(acetylacetonate), titanium(IV) oxide bis (2,2,6,6-tetramethyl-3,5-heptanedionate), titanium(III) tris (2,2,6,6-tetramethyl-3,5-heptanedionato), tris (2,2,6,6-tetramethyl-3,5-heptanedionato)oxotitanium(IV), dichlorobis(2,2,6,6-tetramethyl-3,5-heptanedionato)titanium(IV), titanium(IV) (diisopropoxide)bis(acetylacetonate), di (isopropoxide)bis(2,2,6,6-tetramethyl-3,5-heptanedionato) titanium(IV), zinc(II) bis(acetylacetonate), tetrakis(acetylacetonato)zirconium(IV), zirconium(IV) tetrakis(hexafluoroacetylacetonate), tetrakis(2,2,6,6-tetramethyl-3,5-heptanedionato)zirconium(IV), zirconium(IV) tetrakis(trifluoroacetylacetonate).

**5.** Process according to the preceding claims, **characterized in that** the organometallic complex/compound A mole ratio is between 0.001 and 0.05, preferably between 0.002 and 0.01.

**6.** Process according to the preceding claims, **characterized in that** the cocatalyst is chosen from trialkylamines, heterocyclic aromatic amines, trialkylphosphines, triarylphosphines, trialkyl phosphites, triaryl phosphites and

tetraalkylammonium salts, or mixtures thereof.

**7.** Process according to the preceding claims, **characterized in that** the cocatalyst is used in a proportion of from 1.5 to 3 mol per mole of organometallic complex, preferably 2 mol per mole of organometallic complex.

**8.** Process according to Claim 1, **characterized in that** compound A is chosen from the group of oligomeric or polymeric compounds terminated with at least one cyclocarbonate, preferably at least two cyclocarbonates.

**9.** Process according to the preceding claims, **characterized in that** compound A is a compound corresponding to:

- the general formula A1:

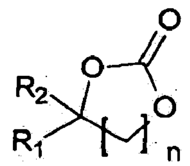

A1

in which:
- n represents an integer equal to 1 or 2,
- $R_1$ represents a hydrogen atom or a linear or branched, saturated or unsaturated $C_{1-6}$ alkyl group, where appropriate substituted with one or more substituents chosen from halogen, hydroxyl, cyano, carboxyl, trifluoromethyl, $C_{1-6}$ alkyl, $C_{1-6}$ alkylamino, $C_{1-6}$ dialkylamino, $C_{1-6}$ alkoxy and $C_{1-6}$ alkylcarboxylate,
- $R_2$ represents a hydrogen atom or a group chosen from: a linear or branched, saturated or unsaturated $C_{1-20}$ alkyl, in which non-neighbouring methylene units may be replaced with radicals -0-, -S-, -S(O)-, $-SO_2-$, -0-C(=O)-, $-N(R^{21})$ -, $-N(R^{21})$-C(=O)-, $-N(R^{21})$ -C(=O)-O-, $-N(R^{21})$-C(=O)-$N(R^{22})$ with $R^{21}$ and $R^{22}$, which may be identical or different, representing hydrogen atoms or radicals chosen from $C_{1-6}$ alkyl or $C_{6-14}$ aryl; a $C_{3-10}$ cycloalkyl; a $C_{6-14}$ aryl; a $C_{3-10}$ heterocycle bearing one or more heteroatoms chosen from N, 0 and S; a $C_{4-13}$ heteroaryl bearing one or more heteroatoms chosen from N, 0 and S; the members of this group being optionally substituted with one or more substituents chosen from halogen, hydroxyl, cyano, carboxyl, trifluoromethyl, $C_{1-6}$ alkyl, $C_{1-6}$ alkylamino, $C_{1-6}$ dialkylamino, $C_{1-6}$ alkoxy and $C_{1-6}$ alkylcarboxylate,
- or, the general formula A2:

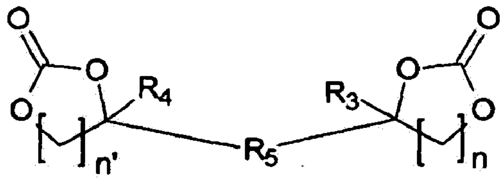

A2

in which:
- n and n', which may be identical or different, represent an integer equal to 1 or 2,
- $R_3$ and $R_4$, which may be identical or different, represent a hydrogen atom or a linear or branched, saturated or unsaturated $C_{1-6}$ alkyl group, where appropriate substituted with one or more substituents chosen from halogen, hydroxyl, cyano, carboxyl, trifluoromethyl, $C_{1-6}$ alkyl, $C_{1-6}$ alkylamino, $C_{1-6}$ dialkylamino, $C_{1-6}$ alkoxy and $C_{1-6}$ alkylcarboxylate,
- $R_5$ represents:

• a direct bond,
• a divalent group chosen from: a linear or branched, saturated or unsaturated $C_{1-20}$ alkylene, in which non-neighbouring methylene units may be replaced with radicals -0-, -S-, -S(O)-, $-SO_2-$, -C(=O)-O-, $-N(R^{21})$ -, $-N(R^{21})$-C(=O)-, $-N(R^{21})$ -C(=O)-O-, -N $(R^{21})$ -C (=O) -N $(R^{22})$ -, with $R^{21}$ and $R^{22}$, which may be identical

or different, representing hydrogen atoms or radicals chosen from $C_{1-6}$ alkyl or $C_{6-14}$ aryl; a $C_{3-10}$ cycloalkylene; a $C_{6-14}$ arylene; a $C_{3-10}$ heterocyclene bearing one or more heteroatoms chosen from N, 0 and S; a $C_{4-13}$ heteroarylene bearing one or more heteroatoms chosen from N, 0 and S; the members of this group being optionally substituted with one or more substituents chosen from halogen, hydroxyl, cyano, carboxyl, trifluoromethyl, $C_{1-6}$ alkyl, $C_{1-6}$ alkylamino, $C_{1-6}$ dialkylamino, $C_{1-6}$ alkoxy and $C_{1-6}$ alkylcarboxylate,

• or a divalent group having the general formula I below:

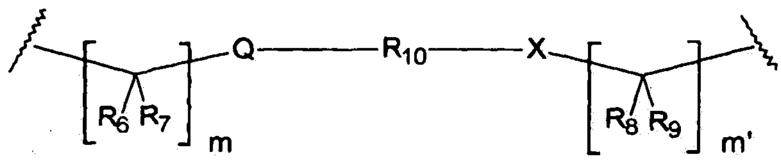

**(I)**

in which:

• m and m', which may be identical or different, denote an integer between 1 and 100,

• $R_6$, $R_7$, $R_8$ and $R_9$, which may be identical or different, represent hydrogen atoms or linear or branched, saturated or unsaturated $C_{1-6}$ alkyl radicals, where appropriate substituted with one or more substituents chosen from halogen, hydroxyl, cyano, carboxyl, trifluoromethyl, $C_{1-6}$ alkyl, $C_{1-6}$ alkylamino, $C_{1-6}$ dialkylamino, $C_{1-6}$ alkoxy and $C_{1-6}$ alkylcarboxylate,

• Q and X, which may be identical or different, represent a group -O-C(=O)-, $-N(R^{21})-$, $-N(R^{21})-C(=O)-$, $-N(R^{21})-C(=O)-O-$, $-N(R^{21})-C(=O)-N(R^{22})-$, with $R^{21}$ and $R^{22}$ as defined previously,

• $R_{10}$ represents a divalent group chosen from: a linear or branched, saturated or unsaturated $C_{1-20}$ alkylene, in which non-neighbouring methylene units may be replaced with radicals -0-, -S-, -S(O)-, $-SO_2-$, -C(=O)-O-, $-N(R^{21})-$, $-N(R^{21})-C(=O)-$, $-N(R^{21})-C(=O)-O-$, $-N(R^{21})-C(=O)-N(R^{22})-$, with $R^{21}$ and $R^{22}$, which may be identical or different, representing hydrogen atoms or radicals chosen from $C_{1-6}$ alkyl or $C_{6-14}$ aryl; a $C_{3-10}$ cycloalkylene; a $C_{6-14}$ arylene; a $C_{3-10}$ heterocyclene bearing one or more heteroatoms chosen from N, 0 and S; a $C_{4-13}$ heteroarylene bearing one or more heteroatoms chosen from N, 0 and S; the members of this divalent group being optionally substituted with one or more substituents chosen from halogen, hydroxyl, cyano, carboxyl, trifluoromethyl, $C_{1-6}$ alkyl, $C_{1-6}$ alkylamino, $C_{1-6}$ dialkylamino, $C_{1-6}$ alkoxy and $C_{1-6}$ alkylcarboxylate,

• or a group corresponding to one of the formulae II or III:

$$-(CH_2-CH_2-O)_t-CH_2-CH_2- \qquad (II),$$

or

$$-C(=O)-CH_2-O-(CH_2-CH_2-O)_{t'}-CH_2-C(=O)- \qquad (III)$$

in which:

- t and t', which may be identical or different, denote an integer between 1 and 100.

**10.** Process according to the preceding claims, **characterized in that** compound A is a compound A3 obtained by:

- reacting 4-hydroxymethyl-1,3-dioxolan-2-one with a compound comprising two or more isocyanate functions,
- the esterification reaction of 4-hydroxymethyl-1,3-dioxolan-2-one with a compound comprising one or more carboxylic acid functions,
- reacting triglycidyl isocyanurate with carbon dioxide, or
- reacting a compound comprising two or more glycidyl ether groups with carbon dioxide.

**11.** Process according to Claim 1, **characterized in that** compound B is chosen from the group of oligomeric or polymeric compounds terminated with at least one reactive unit ($-NH_2$), preferably with at least two reactive units ($-NH_2$).

**12.** Process according to Claim 1 or Claim 11, **characterized in that** the reactive unit (s) ($-NH_2$) is/are borne by saturated

($sp^3$) carbons, which also bear at least one hydrogen, preferably two hydrogens.

**13.** Process according to the preceding claims, **characterized in that** compound B comprising at least one amino ($-NH_2$) reactive unit is chosen from the following list: butylamine; hexylamine; cyclohexylamine; ethanolamine; propanolamine; ethylenediamine; propylenediamine; butylenediamine; pentamethylenediamine; hexamethylenediamine; heptamethylenediamine; tetramethylenediamine; octamethylenediamine; nonamethylenediamine; decamethylenediamine; methyl-2-pentamethylenediamine; undecamethylenediamine; dodecamethylenediamine; xylylenediamine; isophoronediamine; trimethylhexamethylenediamine; 1,2-diaminocyclohexane; 1,4-diaminocyclohexane; 4,4'-diaminocyclohexylmethane; 2-(2-aminoethoxy)ethanol; bis(3-methyl-4-aminocyclohexyl)methane; 2,2-bis(4-aminocyclohexyl)propane; tri(ethaneamine)nitrile; bis(3-aminopropyl)methylamine; 3-amino-1-(methylamino)propane; 3-amino-1-(cyclohexylamino)propane; N-(2-hydroxyethyl)ethylenediamine; 4,7-dioxadecane-1,10-diamine; 4,9-dioxadodecane-1,12-diamine; 7-methyl-4,10-dioxatridecane-1,13-diamine; a polyether monoamine preferably chosen from hydroxy polyethylene glycol amine and methoxy polyethylene glycol amine, a polyether diamine, preferably chosen from a polyoxyethylenediamine and a polyoxypropylenediamine; and/or an oligomer or polymer terminated with at least one amino ($-NH_2$) reactive unit, preferably with two or more amino ($-NH_2$) reactive units, said oligomer or polymer possibly having a polyamide, polyether and/or polyester backbone.

**14.** Process according to the preceding claims, **characterized in that** compound A and compound B are mixed so that the mole ratio of compound B to compound A is between 0.5 and 2, preferably equal to 1.

**15.** Process according to the preceding claims, **characterized in that** compound A and compound B are premixed with a plasticizer before being placed in contact with the catalyst, which catalyst comprises at least the organometallic complex and the cocatalyst.

**16.** Use of a catalyst comprising at least one organometallic complex and a cocatalyst chosen from the group of Lewis bases and/or tetraalkylammonium salts, for catalyzing the condensation reaction between a compound A comprizing at least one cyclocarbonate reactive unit and a compound B comprising at least one amino ($-NH_2$) reactive unit.

*FIG. 1 :*

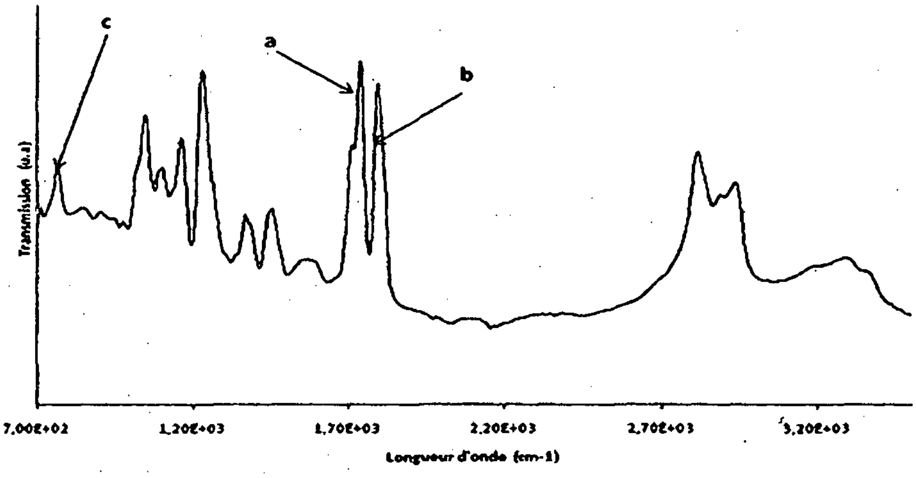


a: $\nu_{C=O(uréthane)}$ ; b: $\nu_{C=O(cyclocarbonate)}$ ; c: $\nu_{csp2-H\ (cyclocarbonate)}$

*FIG. 2 :*

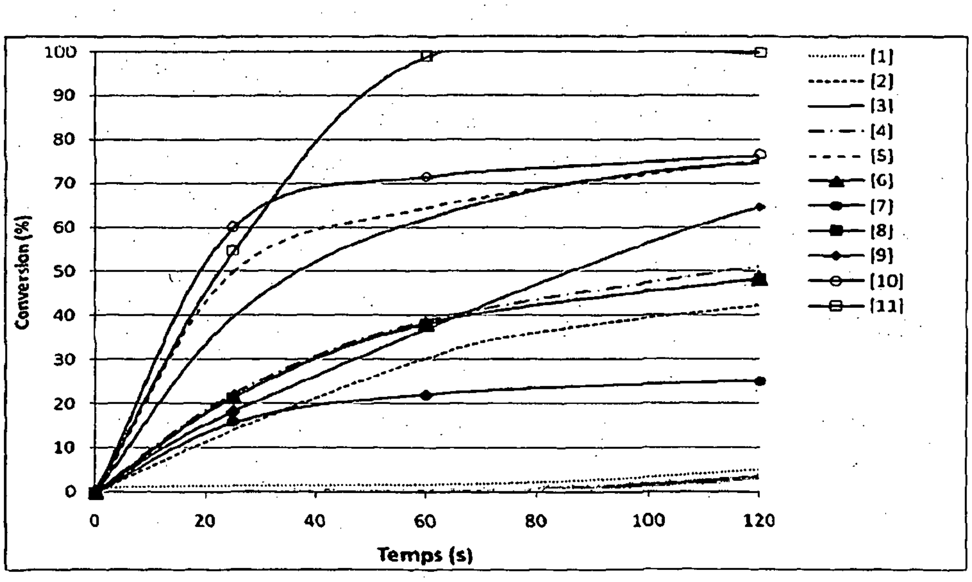

*FIG. 3*

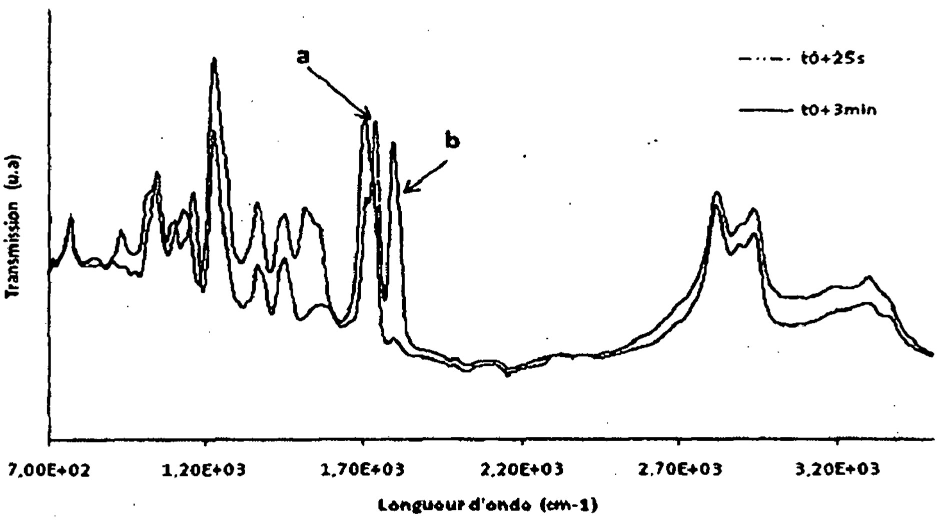


a: νC=O(uréthane) ; b: νC=O(cyclocarbonate)

*FIG. 4.*

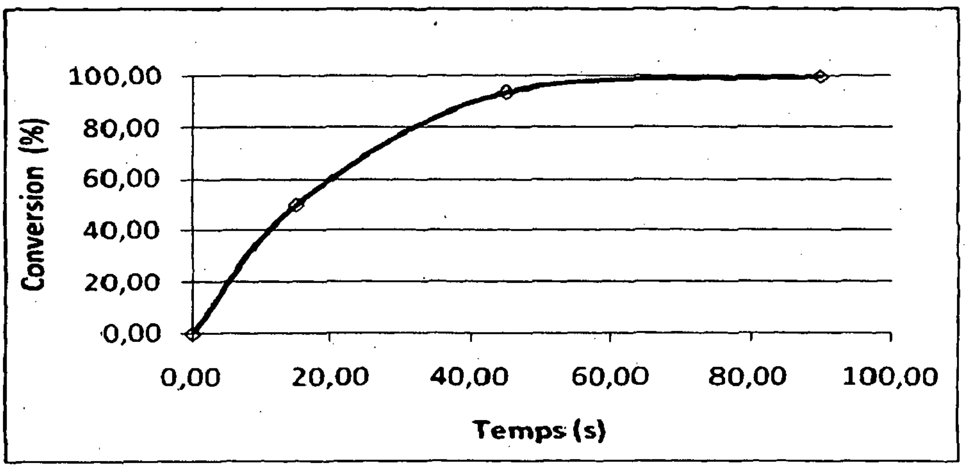

FIG. 5 :

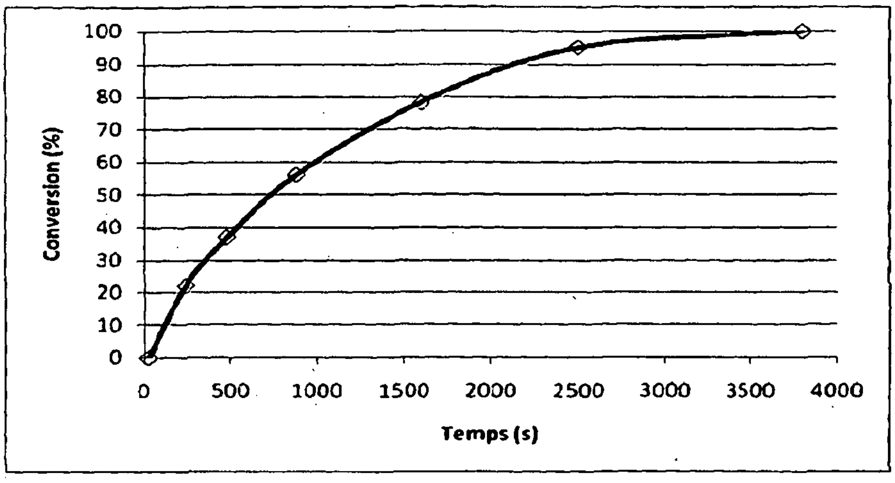
Conversion (%)
100
90
80
70
60
50
40
30
20
10
0
0
500
1000
1500
2000
2500
3000
3500
4000
Temps (s)

FIG. 6:

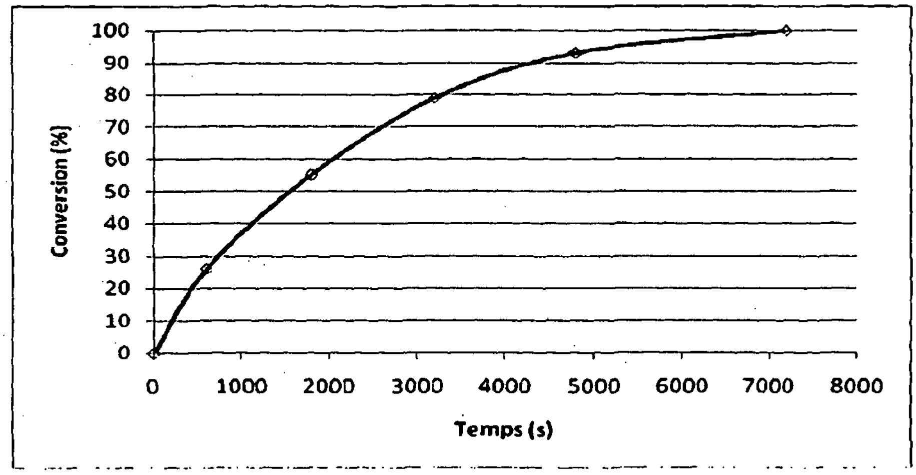
Conversion (%)
100
90
80
70
60
50
40
30
20
10
0
0
1000
2000
3000
4000
5000
6000
7000
8000
Temps (s)

FIG. 7 :

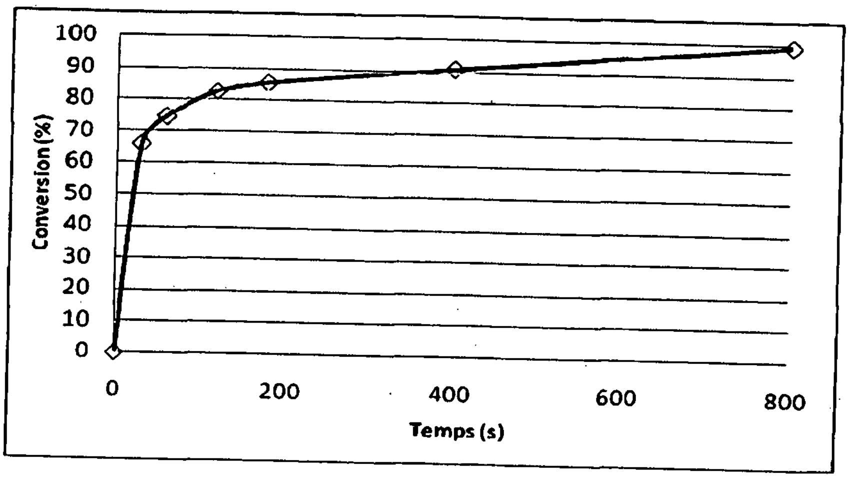
100
90
80
70
60
50
40
30
20
10
0
Conversion (%)
0
200
400
600
800
Temps (s)

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- US 5340889 A, Crawford
- US 2802022 A, Groszos
- US 4758615 A, Engel
- US 6120905 A, Figovsky
- US 2935494 A, Whelan
- US 3072613 A, Whelan
- EP 1070733 A, POLYMATE LTD
- US 5055542 A, Honel
- US 5132458 A, Honel
- US 5977262 A, Anderson
- WO 2003066580 A
- WO 2005016993 A
- US 4268684 A, Arthur G.
- EP 0065026 A
- US 7232877 B, Figovsky
- US 5175231 A, Rappoport
- US 6495637 B, Rappoport
- US 6410127 B
- US 5391826 A, Speranza
- US 5908894 A, Genz


### Littérature non-brevet citée dans la description


- **OCHIA, B ; SATOH, Y ; ENDO, T.** *J, Polym. Sci. Part A : Polym Chem.,* 2001, vol. 39, 4091
- *Journal of Medeleev Chemical Society,* 1988, vol. 33 (3), 31-36
- **R.C. MEHROTRA.** Metal Beta-diketonates and Allied Derivatives. Academic Press, 1978
- Metal β-Ketoenolate Complexes. **FACKLER, J.P., JR.** Progress in Inorganic Chemistry. Interscience, 1966, vol. 7, 471